# EUROPEAN PATENT SPECIFICATION

(11) **EP 1 839 554 B1**
(45) Date of publication and mention of the grant of the patent: **25.12.2013**
(21) Application number: 06712073.3
(22) Date of filing: 20.01.2006
(51) Int. Cl.: A61B 17/34, A61B 1/00, A61B 19/00, B41M 5/26, A61B 17/00

(54) **ENDOSCOPE, MEDICAL INSTRUMENT FOR ENDOSCOPE AND METHOD APPLYING MARKINGS THERETO**
ENDOSKOP, MEDIZINISCHE VORRICHTUNG FÜR EIN ENDOSKOP UND VERFAHREN HIERFÜR ZUM AUFBRINGEN VON KENNZEICHNUNGEN
ENDOSCOPE, APPAREIL MEDICAL POUR L'ENDOSCOPE ET PROCEDE D'APPLICATION DE MARQUAGE DE CELLUI-CI

(30) Priority: 21.01.2005 JP 2005014292; 09.02.2005 JP 2005033161; 15.02.2005 JP 2005038017
(43) Date of publication of application: 03.10.2007
(73) Proprietor: Olympus Corporation, Tokyo 151-0072 (JP); OLYMPUS MEDICAL SYSTEMS CORP., Tokyo 151-0072 (JP)
(72) Inventor: NAKAMURA, Mitsuhiro, c/o Int. Prop. Support Department, Hachioji-shi, Tokyo 192-8512 (JP); MATSUMOTO, Jun, c/o Int. Prop. Support Department, Hachioji-shi, Tokyo 192-8512 (JP); NAKAMURA, Takeaki, c/o Int. Prop. Support Department, Hachioji-shi, Tokyo 192-8512 (JP); YAMADA, Noboru, c/o Int. Prop. Support Department, Hachioji-shi, Tokyo 192-8512 (JP)
(74) Representative: von Hellfeld, Axel
(86) International application number: PCT/JP2006/300848
(87) International publication number: WO 2006/077965

(56) References cited:
- EP-A1- 0 708 147
- WO-A1-2004/050767
- DE-A1- 10 359 997
- GB-A- 2 352 824
- JP-A- 08 243 071
- JP-A- 2002 136 600
- JP-A- 2002 188 016
- JP-A- 2002 273 832
- JP-A- 2003 277 570
- JP-A- 2004 195 030
- JP-A- 2004 195 031
- JP-A- 2004 321 395
- US-A- 5 350 792
- US-A- 5 489 639
- US-A- 5 719 372
- US-A1- 2004 132 892
- US-B1- 6 168 853

## Description

### Technical Field

This invention relates to an endoscope to be employed generally for the observation, examination, diagnosis and treatment of body cavities of human body, to a medical instrument for the endoscope, and to a method of applying markings to the endoscope and the medical instrument for endoscope.

### Background Art

The endoscope is generally designed to be inserted into a body cavity of human body in order to perform the observation, examination, diagnosis and treatment of a subject. The endoscope is generally constituted by an insertion portion which is designed to be inserted into a body cavity, and a manipulation portion for manipulating this insertion portion. The insertion portion is formed of a cylindrical flexible tube. This insertion portion is provided with an indication such as a distance marker for enabling an operator to determine the length that has been inserted into the body cavity. The manipulation portion is provided with, for example, the name of manufacturer, logotype, product name, and, additionally, the designations representing the functions of button and lever.

As for the method for applying these markings to an endoscope, a printing method using ink has been generally employed, examples of the printing method including inking, pad printing, screen printing, brush printing, etc. As for the kinds of ink to be used in this case, they include, for example, an urethane-based ink and an epoxy-based ink. For example, JP Patent Publication 61-241184 discloses a printing method using a photocurable ink. JP-A 2003-88469 (KOKAI) discloses a printing method using a fluorocarbon-based ink.

The endoscope is generally sterilized according to the following sterilization methods. They include, for example, a sterilization method employing a glutaraldehyde-based sterilizing liquid, a sterilizing liquid containing peracetic acid or a sterilizing liquid containing hydrogen peroxide; a sterilization method employing hydrogen peroxide and low temperature plasma; and an autoclave sterilization method.

When the endoscope is repeatedly subjected to these sterilization methods, the discoloration or blurring of printed marks or the peel-off of printed marks fro base resin is caused to generate if the printing is performed using ink. As a result, it may become difficult to identify the printed indications, letters, etc. There are known base resins which are resistive to the repeated sterilizations. Most of these base resins however are generally poor in adhesive strength, or poor in adhesion to ink, permitting the ink to easily peel off.

As for the underlying layer on which printing is to be performed using ink in the endoscope, a polyolefin-based resin or a fluorinated resin is employed for example. These resins are poor in adhesion to ink and hence it is difficult to perform printing on these resins.

The endoscope is frequently scrubbed on the occasion of washing it. Therefore, there is a problem that the printed marks are blurred by the washing of endoscope.

Many kinds of ink contain an organic solvent. Therefore, many kinds of ink are accompanied with environmental problem and a problem of safety to workers. In the working process for performing the printing using ink, there are many problems that it requires, in order to execute the printing work, auxiliary facilities or equipments such as a drying chamber, a UV illumination system, etc., and that a large number of printing plates are required to be prepared taking much time in the preparation thereof.

The application of marks to recent industrial products is currently performed by a method wherein marks are put on the products by the irradiation of laser beam. This method is frequently employed in the printing of lot number or ID number for instance.

JP-A 8-131448 (KOKAI) discloses the printing of maker name, type number, etc., by making use of laser marking in a treating apparatus for endoscope. JP-A 2004-195030 (KOKAI) discloses a method for performing laser marking to a medical instrument for endoscope so as to obtain a colored matter which can be hardly degraded.

JP Patent Publication 62-59663 (KOKAI) discloses a method for applying a marking to a resin through the irradiation of laser beam. In this publication, there is disclosed the mixing of plastics with a filler which can be changed in color as it is irradiated with an energy beam, the resultant mixture being subsequently printed on a resin. JP Patent Publication 61-41320 (KOKAI) discloses a method of marking wherein laser beam is applied to the surface of synthesized substance comprising a dye and a silicon-containing compound or silicon.

DE 103 59 997 A1 discloses a component for an endoscope comprising an external surface having a graduate scale. The scale is obtained through a color developer TiNₓOₓ which is irradiated through laser light.

JP 2002 273832 A discloses a plastic multilayered sheet molded object for applying laser marking on a plastic multilayered sheet.

WO 2004/050767 A1 discloses a laser writable composition with a polymeric laser light absorber dispersed in a matrix polymer.

US 2004/0132892 A1 discloses a white coloring laser-marking thermoplastic resin composition.

US 6,168,853 B1 discloses a method for laser marking phosphorescent plastic articles.

GB 2 352 824 A discloses a laser markable material comprising TiO₂ pigmented thermoplastic elastomer.

JP 2002 188016 A discloses a resin composition for laser marking with transparency. A color powder using based on TiNₓOₓ is used.

EP 0 708 147 A1 discloses a laser marking resin composition.

US 5,489,639 discloses the use of copper salts for laser marking of thermoplastic compositions.

US 5,350,972 discloses a pigment-containing plastic molding composition.

### Disclosure of Invention

The marking method employing laser marking to obtain a colored matter is considerably inferior in terms of contrast and visibility as compared with a printed matter created using ink. Therefore, this marking method is accompanied with a problem that the markings applied cannot be easily recognized.

The endoscope is provided, at various portions thereof, with various kinds of markings which are applied by making use of a printing method using an ink. For example, the insertion portion is provided with a graduation for enabling an operator to recognize the length that has been inserted into the body cavity. The manipulation portion is provided with, for example, the name (logotype) of the manufacturer of the endoscope, and the product name. If printing is applied to various portions of endoscope by making use of ink, a large number of steps are required in the manufacture of endoscope.

For example, when the graduation and the name (logotype) of the manufacturer are printed on insertion portion, it is possible to reduce the steps of printing. The graduation is printed in the circumferential direction (radial direction) in perpendicular to the axis of insertion portion. The name (logotype) of the manufacturer is printed along the axial direction of the insertion portion. Therefore, in viewpoint of design, the graduation can be hardly mistaken for the name (logotype) of the manufacturer.

According to a first aspect of the present invention, there is provided an endoscope and a medical instrument for endoscope, comprising: the endoscope and the medical instrument are at least partially formed of thermoplastic resin, thermosetting resin or rubber and are provided with a portion which is formed of thermoplastic resin, thermosetting resin or rubber each containing a colorant or a filler; and that; the endoscope and the medical instrument are provided with a marking portion indicating marks including letters and symbols which are developed through an irradiation of said portion with a pulse laser beam of YAG or YVO₄ having a wavelength of 355, 532 or 1064 nm.

According to a second aspect of the present invention, an endoscope and a medical instrument for endoscope, comprising: the endoscope and the medical instrument are at least partially formed of thermoplastic resin, thermosetting resin, rubber or thermoplastic elastomer and are provided with a portion which is formed of thermoplastic resin, thermosetting resin, rubber or thermoplastic elastomer each containing 0.001 to 20 parts by weight of carbon black having an average particle diameter of 10-80 nm as a colorant/color-developing agent/filler; and that; the endoscope and the medical instrument are provided with a marking portion indicating marks including letters and symbols which are developed through an irradiation of said portion containing the carbon black with a pulse laser beam of YAG or YVO₄ having a wavelength of 355, 532 or 1064 nm.

According to a second aspect of the present invention, an endoscope and a medical instrument for endoscope, which comprise an inserting flexible tube composed of a spiral tube, a mesh tube and a skin which are concentrically laminated; wherein the skin is provided thereon with a first indicator for determining a length inserted of the inserting flexible tube is formed along a line perpendicular to the axial direction of the inserting flexible tube and with a second indicator which differs in features from the first indicator.

### Brief Description of Drawings

FIG. 1 is a perspective view schematically illustrating the construction of a laser marking apparatus to be used in a first embodiment of the endoscope and the medical instrument for endoscope according to the present invention;
FIG. 2 is a diagram illustrating the formation of marking by the effects of laser marking which is designed to be applied to the endoscope and the medical instrument for endoscope;
FIG. 3 is a diagram illustrating the scanning of pulse laser beam for forming a marking to be applied to the endoscope and the medical instrument for endoscope;
FIG. 4 is a diagram showing the results of marking for setting the marking conditions of marking portion to be applied to the endoscope and the medical instrument for endoscope;
FIG. 5 is a diagram showing the results of marking for setting the marking conditions of marking portion to be applied to the endoscope and the medical instrument for endoscope;
FIG. 6 is a perspective view showing an external appearance of the endoscope illustrating each of marking portions to be put on the endoscope;
FIG. 7A is a diagram showing a marking that has been put on the endoscope and the medical instrument for endoscope;
FIG. 7B is a diagram showing a marking that has been put on the endoscope and the medical instrument for endoscope according to the prior art;
FIG. 8 is a perspective view showing an external appearance of the endoscope illustrating each of marking portions to be put on the endoscope according to a second embodiment of the endoscope and the medical instrument for endoscope of the present invention;
FIG. 9 is a longitudinal sectional view of the insertion portion made of a flexible tube of endoscope attached with a marking;
FIG. 10A is a diagram showing a marking that has been put on the endoscope according to the present invention;
FIG. 10B is a diagram showing a marking that has been put on the endoscope and the medical instrument for endoscope according to the prior art;
FIG. 11 is a longitudinal sectional view of one modified example of the insertion portion made of a flexible tube of endoscope attached with a marking;
FIG. 12 is a perspective view showing an external appearance of the endoscope having marking portions attached thereto according to a third embodiment of the method of applying markings according to the present invention;
FIG. 13 is a perspective view showing an external appearance of the insertion portion made of a flexible tube, on which a graduation and the name of manufacturer are attached to the endoscope;
FIG. 14 is a perspective view schematically illustrating the construction of a laser marking apparatus for applying markings to the endoscope; and
FIG. 15 is a diagram showing a state of focusing of the converging optical system of the laser marking apparatus.

Best Mode for Carrying Out the Invention Next, a first embodiment of the present invention will be explained with reference to drawings.

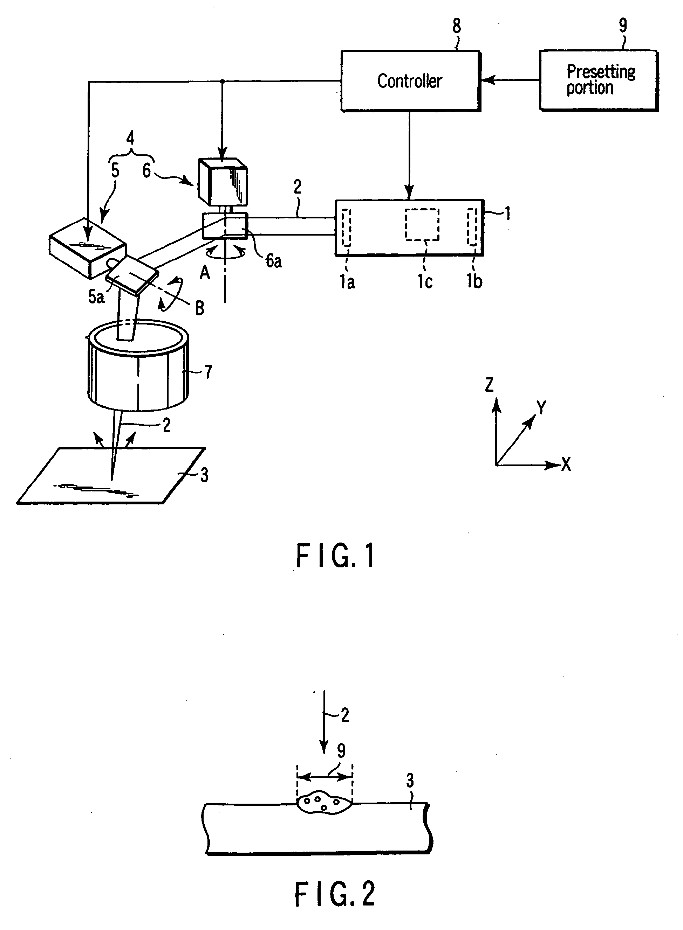

FIG. 1 shows a perspective view schematically illustrating the construction of a laser marking apparatus to be used for applying markings by way of laser marking to the endoscope and the medical instrument for endoscope. A Q-switch pulse laser beam source (hereinafter referred to as a pulse laser beam source) 1 is enabled to output a pulse laser beam 2 having a wavelength of 1064 nm for instance. In this pulse laser beam source 1, Nd:YVO₄ laser (hereinafter referred to as YVO₄ laser) is employed for example. Alternatively, Nd:YAG laser (hereinafter referred to as YAG laser) which is capable of creating a pulse laser beam 2 having a wavelength of 1064 nm may be employed for this pulse laser beam source 1. These YVO₄ laser and YAG laser are enabled to output a pulse laser beam 2 having a wavelength of 355 nm and a pulse laser beam 2 having a wavelength of 532 nm, respectively, as they are subjected to a half-wave conversion.

The YAG laser is caused to change considerably in peak output value depending on the scanning speed thereof. On the other hand, the YVO₄ laser is enabled to exhibit only small changes in peak output value even if the scanning speed thereof is changed. The YAG laser is generally of multiplex mode. On the other hand, the YVO₄ laser is of single mode. The single mode is more advantageous in that the markings to be created by means of laser marking are more excellent in contrast and visibility.

The endoscope and the medical instrument for endoscope 3 are adapted to be subjected to laser marking. Thus, markings representing letters and symbols including marks can be put or depicted on the surface of the endoscope and the medical instrument for endoscope. These markings can be depicted by means of not only the YVO₄ laser but also the YAG laser. Especially, it is more preferable to employ the YVO₄ laser of single mode.

The endoscope and the medical instrument for endoscope 3 are at least partially formed of thermoplastic resin, thermosetting resin or rubber and are provided with a portion which is formed of thermoplastic resin, thermosetting resin or rubber each containing a colorant or a filler.

As for the resin to be employed for manufacturing the endoscope, it is possible to employ various kinds of engineering plastics, various kinds of super engineering plastics, and various kinds of thermoplastic resin. More specifically, it is possible to employ thermoplastic resins including polyolefin, polyethylene, polypropylene, polycarbonate, acrylonitrile-butadiene-styrene, polystyrene, polyoxymethylene acetal, polyamide nylon, polybutylene terephthalate, polysulfone, polyether sulfone, polyurethane, polyester, Noryl, polyether imide, polyether nitrile, polyetherether ketone, polyimide, polyphthalamide, polyphenylene ether, fluorinated resin such as polytetrafluoroethylene, thermoplastic polyurethane, etc.

As for the colorant and filler, it is possible to employ at least one kind of material selected from carbon black, calcium carbonate, black iron oxide, titanium black, titanium dioxide, etc.

On the occasion of applying laser marking to the thermoplastic resin, thermosetting resin or rubber, the wavelength of the pulse laser beam 2 to be emitted from the pulse laser beam source 1 for realizing the laser marking may be 1064, 532 or 355 nm. The colorant and filler should be selected from those having a strong absorption band to the wavelengths of visible region and ultraviolet region. By making use of these colorant and filler, pulse laser beam 2 having a wavelength of 532 or 355 nm may be suitably employed in applying laser marking to the surface of thermoplastic resin, thermosetting resin or rubber at a lower laser output and without giving damage to the endoscope and the medical instrument for endoscope 3. It is also possible to obtain an excellent marking by irradiating a pulse laser beam having a wavelength of 266 nm which can be obtained through the conversion in wavelength of 1064 nm into a quarter.

The pulse laser beam source 1 is equipped with an output mirror 1a and a high-reflection mirror 1b. A Q-switch 1c is interposed between the output mirror 1a and the high-reflection mirror 1b. This Q-switch 1c is enabled to perform on-off control action. Due to this on-off control action of the Q-switch 1c, a pulse laser beam 2 is permitted to emit from the pulse laser beam source 1.

An XY scanner 4 is proved on the optical line of the pulse laser beam 2 to be output from the pulse laser beam source 1. The XY scanner 4 is constituted by an X-axis scanner 5 and a Y-axis scanner 6. The Y-axis scanner 6 is actuated in such a manner that a Y-axis scanning mirror 6a is swung in the direction of allow "A", thereby enabling the pulse laser beam 2 emitted from the pulse laser beam source 1 to scan in the direction of Y-axis. The X-axis scanner 5 is actuated in such a manner that an X-axis scanning mirror 5a is swung in the direction of allow "B", thereby enabling the pulse laser beam 2 being scanned in the direction of Y-axis by the Y-axis scanner 6 to scan in the direction of X-axis.

A fθ lens 7 is proved on the optical line of the pulse laser beam 2 that has been scanned in the XY-axes by the XY scanner 4. This fθ lens 7 is designed to converge the pulse laser beam 2 that has been scanned in the XY-axes by the XY scanner 4 on the surface of the endoscope and the medical instrument for endoscope 3, thereby creating a spot light thereon.

The controller 8 is designed to perform a plurality of functions including the initiation and termination of the laser output operation of the pulse laser beam source 1, the control of the laser output power of the pulse laser beam source 1, the control of the switching action of the Q-switch 1c, the control of the scanning action of each of the X-axis scanner 5 and the Y-axis scanner 6, etc.

In order to enhance the contrast and visibility of the marking portions depicted by means of the laser marking on the surface of the pulse laser beam source 1, it is necessary to enhance the density of the color-developing portion in the coated portion of the marking that can be created by the irradiation of the pulse laser beam 2. The density of this color-developing portion is influenced by the spot size of the pulse laser beam 2, by the intervals of hatching of the pulse laser beam 2, by the Q-switching frequency Qf of the pulse laser beam 2 and by the scanning speed Sp of the pulse laser beam 2.

When the pulse laser beam 2 is irradiated onto the endoscope and the medical instrument for endoscope 3 as shown in FIG. 2, a portion of the endoscope and the medical instrument for endoscope 3 which has been irradiated with the pulse laser beam 2 is caused to discolored into a whitish color. Thus, this discolored portion becomes a color-developed portion 9.

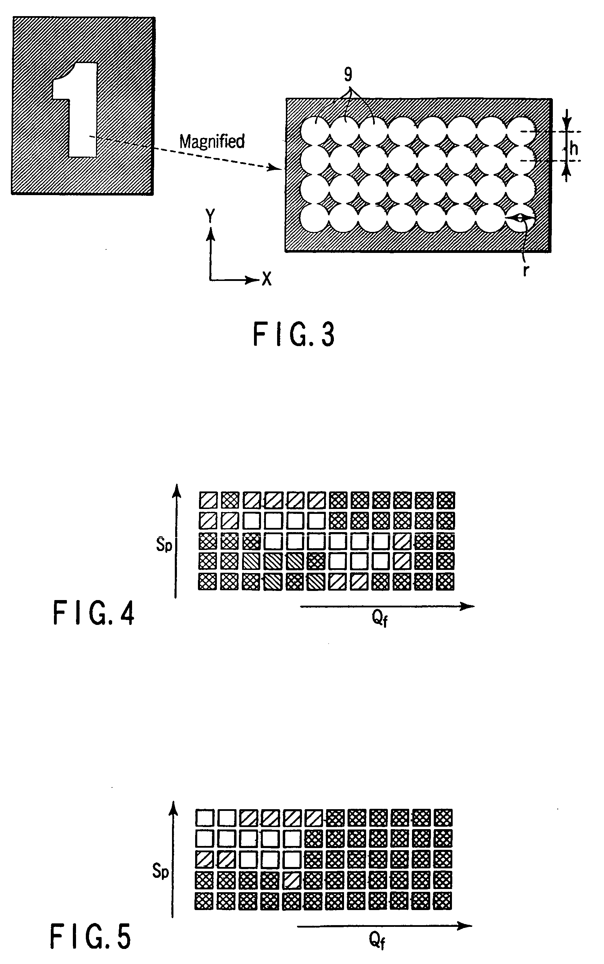

Next, there will be explained a method for forming a marking portion representing letters or symbols such as a number "1" for example as shown in FIG. 3. In this case, the pulse laser beam 2 is scanned in the directions of the X- and Y-axes every pulse as shown in an enlarged portion of FIG. 3. As a result, a plurality of color-developed portions 9 each generated by the pulse laser beam 2 of every pulse are formed in the directions of X- and Y-axes. In this case, neighboring color-developed portions 9 are prevented from being superimposed with each other.

If it is desired to enhance the density of each of the color-developed portions 9 to be created through the irradiation of the pulse laser beam 2, the marking conditions such as the spot diameter r of pulse laser beam 2, the intervals of hatching h of pulse laser beam 2, the Q-switching frequency Qf of pulse laser beam 2, the scanning speed Sp of pulse laser beam 2, etc., are required to be set suitably by taking into account the specific material constituting each of the endoscope and the medical instrument for endoscope 3.

Next, the procedure of setting these marking conditions will be explained. First of all, the output of pulse laser beam 2, the intervals of hatching h of pulse laser beam 2, the spot diameter r of pulse laser beam 2, the size of fθ lens 7, the number of steps are set as marking conditions. FIGS. 4 and 5 show the results obtained from the marking of a symbol "□", in which the scanning speed Sp of pulse laser beam 2 and the Q-switching frequency Qf of pulse laser beam 2 are respectively changed.

The results of marking vary depending on the material constituting each of the endoscope and the medical instrument for endoscope 3. Specifically, the material constituting each of the endoscope and the medical instrument for endoscope 3 is formed from a combination of a thermoplastic resin, a thermosetting resin or rubber with at least one kind of material selected from carbon black, calcium carbonate, black iron oxide, titanium black and titanium dioxide. As for the result of marking, it is not limited to those shown in FIGS. 4 and 5, but it can be a plurality of different kinds of marking that can be achieved by changing the marking conditions and the material constituting the endoscope and the medical instrument for endoscope 3.

As seen from the results of marking, the density of white color developed of each of symbols "□" can be caused to vary by changing the scanning speed Sp of pulse laser beam 2 and the Q-switching frequency Qf of pulse laser beam 2. Based on this density of white color developed of each of symbols "□", the quality of contrast and visibility of the symbols are determined. Among these symbols of white color thus developed, the most excellent symbol "□" is selected.

In this manner, optimum marking conditions for improving the contrast and visibility of the marking portion are determined on the basis of the results of marking for each kind of materials constituting the endoscope and the medical instrument for endoscope 3. For example, marking conditions which make it possible to secure, for example, "3" or more in contrast value between the color-developed portions 9 formed on the surface of the endoscope and the medical instrument for endoscope 3 and the underlying substrate thereof; 60 or more in brightness; -20 to +20 in shade; and -10 to +10 in chroma are determined.

As a result, it has been determined that the marking conditions should preferably be adjusted as follows. For example, the spot diameter r of pulse laser beam 2 should preferably be confined within the range of 5-100 µm, the intervals of hatching h of pulse laser beam 2 should preferably be confined within the range of 1-80 µm, the Q-switching frequency Qf of pulse laser beam 2 should preferably be confined within the range of 0.1-100 kHz, and the scanning speed Sp of pulse laser beam 2 should preferably be confined within the range of 1-3000 mm/sec. More preferably, the spot diameter r of pulse laser beam 2 should be limited to not more than 40 µm, the intervals of hatching h of pulse laser beam 2 should be 30 µm, the Q-switching frequency Qf of pulse laser beam 2 should be 30 kHz, and the scanning speed Sp of pulse laser beam 2 should be confined within the range of 2-3000 mm/sec.

The peak output of the pulse laser beam 2 should preferably be as large as possible. However, in order to enhance the contrast and visibility of the marking portion in relation with the materials constituting the endoscope and the medical instrument for endoscope 3, the peak output of the pulse laser beam 2 should preferably be adjusted within the range of 0.1-50W. Meanwhile, an average output of the pulse laser beam 2 is confined within the range of 0.1-50W.

The pulse width of the pulse laser beam 2 to be irradiated onto the surfaces of the endoscope and the medical instrument for endoscope 3 should preferably be as small as possible in order to create marking portion which is further improved in contrast and visibility. Therefore, the pulse width of the pulse laser beam 2 should preferably be adjusted within the range of 0.1-200 ns. It is especially preferable to confine the pulse width of the pulse laser beam 2 to 10 ns.

The controller 8 is designed to control the switching operation of the Q-switch 1c, the scanning speed Sp of each of the X-axis scanner 5 and the Y-axis scanner 6 of the XY scanner 4, and the Q-switching frequency Qf of the Q-switch 1c so as to realize the predetermined marking conditions which are set in advance with respect to the spot diameter r of pulse laser beam 2 (=5-100 µm), the intervals of hatching h of pulse laser beam 2 (=1-80 µm), the Q-switching frequency Qf of pulse laser beam 2 (0.1-100 kHz), and the scanning speed Sp of pulse laser beam 2 (1-3000 mm/sec).

The presetting section 9 is designed to transmit to the controller 8 preset values with respect to the spot diameter r of pulse laser beam 2, the intervals of hatching h of pulse laser beam 2, the Q-switching frequency Qf of pulse laser beam 2, and the scanning speed Sp of pulse laser beam 2.

Next, the process of laser marking by making use of the laser marking apparatus constructed as described above will be explained.

The marking conditions are transmitted from the presetting section 9 to the controller 8. The marking conditions are preset, for example, to 5-100 µm as the spot diameter r of pulse laser beam 2, to 1-80 µm as the intervals of hatching h of pulse laser beam 2, to 0.1-100 kHz as the Q-switching frequency Qf of pulse laser beam 2, and to 1-3000 mm/sec as the scanning speed Sp of pulse laser beam 2. More specifically, the marking conditions are preset to not more than 40 µm as the spot diameter r of pulse laser beam 2, to 30 µm as the intervals of hatching h of pulse laser beam 2, to 30 kHz as the Q-switching frequency Qf of pulse laser beam 2, and to 2-3000 mm/sec as the scanning speed Sp of pulse laser beam 2. The scanning speed Sp of pulse laser beam 2 may be set to 2000 mm/sec.

By means of the controller 8, the switching operation of the Q-switch 1c, the scanning speed Sp of each of the X-axis scanner 5 and the Y-axis scanner 6 and the Q-switching frequency Qf of the Q-switch 1c are respectively controlled according to the preset marking conditions including the intervals of hatching h of pulse laser beam 2, the Q-switching frequency Qf of pulse laser beam 2 and the scanning speed Sp of pulse laser beam 2.

The pulse laser beam 2 is emitted from the pulse laser beam source 1. The pulse laser beam 2 is transmitted to the fθ lens 7. The pulse laser beam 2 transmitted to the fθ lens 7 is then scanned as a spot light over the surfaces of endoscope and the medical instrument for endoscope 3. The spot diameter r of pulse laser beam 2 is converged by means of this fθ lens 7 to a diameter of 5-100 µm, especially to not more than 40 µm.

As the pulse laser beam 2 is irradiated onto the surface of the endoscope and the medical instrument for endoscope 3, the region of the endoscope and the medical instrument for endoscope 3 that has been irradiated with the pulse laser beam 2 is discolored into whitish color, thus creating a color-developed portions 9.

Additionally, the pulse laser beam 2 is scanned in the directions of the X- and Y-axes every pulse. As a result, a plurality of color-developed portions 9 thus created are formed in the directions of X- and Y-axes as shown in FIG. 3 with neighboring color-developed portions 9 being prevented from being superimposed with each other. In this case, in order to enhance the density of each of the color-developed portions 9 to be created through the irradiation of the pulse laser beam 2, the intervals of hatching h of pulse laser beam 2 are confined to 1-80 µm as shown in FIG. 3. More specifically, the spot diameter r of pulse laser beam 2 is confined to not more than 40 µm and the intervals of hatching h of pulse laser beam 2 are confined to 30 µm. As a result, a marking portion comprising letters, symbols, etc., can be depicted on the surface of the endoscope and the medical instrument for endoscope 3.

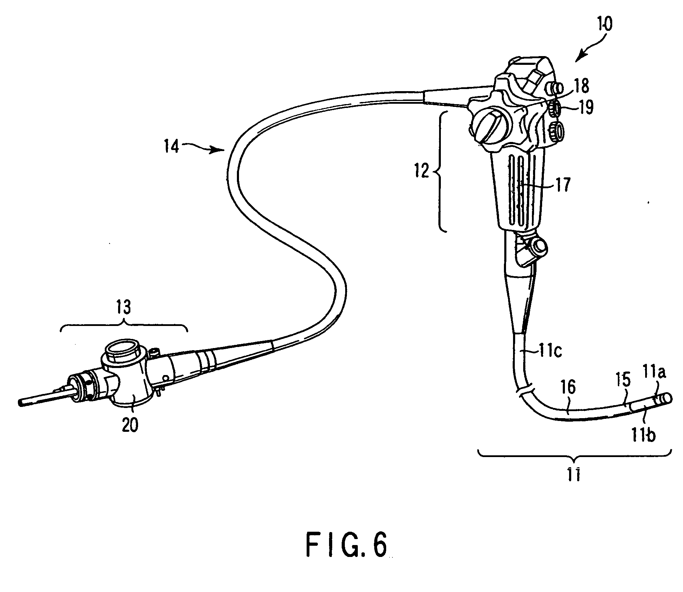

FIG. 6 shows an external appearance of the endoscope 10. This endoscope 10 is constituted by an inserting flexible tube 11, a manipulation portion 12, a connector portion 13, and a manipulation portion/connector portion connecting tube 14. The inserting flexible tube 11 is designed to be inserted into a body cavity of human body or a subject. This inserting flexible tube 11 is constituted by a distal end portion 11a, a curved portion 11b and a soft portion 11c. The distal end portion 11a is provided with an observation window and an illumination window. The manipulation portion 12 is designed to manipulate the inserting flexible tube 11. This manipulation portion 12 is equipped with a UD (up/down) angle knob, a UD angle-canceling knob, an RL (right/left directions) angle knob, an RL angle-canceling knob, a suction button, an air/water feeding button, etc.

The inserting flexible tube 11, manipulation portion 12 and connector portion 13 of the endoscope 10 are respectively provided with marking portions 15-20 which can be applied thereto by means of laser marking. For example, the inserting flexible tube 11 is provided with a marking portion 15 indicating a white indicator line, and with a marking portion 16 indicating a logotype. The white indicator line 15 is provided for determining the depth of insertion of the inserting flexible tube 11 into the body cavity.

The manipulation portion 12 is provided with a marking portion 17 indicating the type and name of the endoscope 10, with a marking portion 18 indicating various kinds of angles such as a UD angle knob, a UD angle-canceling knob, an RL angle knob and an RL angle-canceling knob, and with a marking portion 19 indicating various kinds of buttons such as a suction button, an air/water feeding button.

The connector portion 13 is designed to be connected with a signal processor for images, etc. This connector portion 13 is provided with a marking portion 20 indicating, for example, the name of manufacturer and logotype.

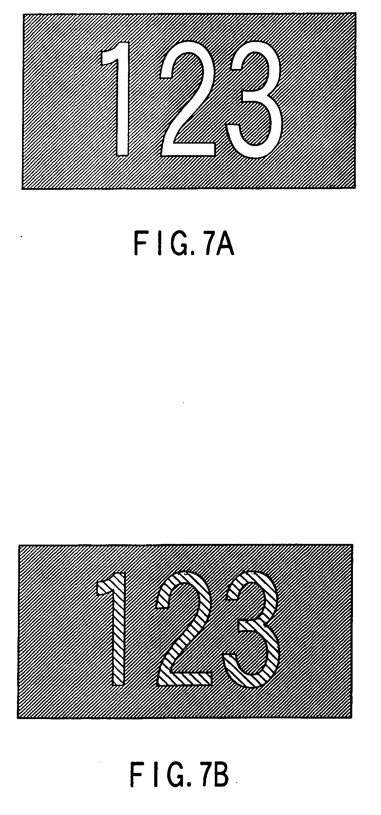

FIGS. 7A and 7B illustrate the comparison between the present invention and the prior art. Namely, FIG. 7A shows one example of the marking portion 15 applied to the endoscope 10 of the present invention. FIG. 7B shows a marking portion which was applied to the endoscope 10 by means of the conventional laser marking. It will be recognized from the comparison of these marking portions that the marking portion 15 applied to the endoscope 10 of the present invention was more excellent in contrast and visibility as compared with the conventional marking portion.

Next, Examples 1-6, an example according to the prior art, and Comparative Example will be explained with reference to Table 1.

**Table 1**

| | | Conventional example 1 | Comparative example | Example 1 | Example 2 | Example 3 | Example 4 | Example 5 | Example 6 |
|---|---|---|---|---|---|---|---|---|---|
| Marking method | | Ink | Laser | | | | | | |
| Resin composition | Thermoplastic elastomer | Polyester 100 | | | | | | | |
| | Carbon black | 1 | | 1 | | | | | |
| | Calcium carbonate | | | 0.1 | | | | | |
| | Black iron oxide | | | 0.1 | | | | | |
| | Titanium black | | 10 | | | | | | |
| | Titanium dioxide | | | 1 | | | | | |
| Conditions for laser beam | Laser species | | YAG | YAG | | | YV0₄ | | |
| | Wavelength (rim) | | 1064 | 1064 | 532 | 355 | 1064 | 532 | 355 |
| | Filling hatching intervals (µm) | | 100 | 30 | 30 | 30 | 30 | 30 | 30 |
| | Laser spot diameter (µm) | | 100 | 80 | 30 | 10 | 30 | 10 | 10 |
| | Laser peak output (kW) | | 30 | 100 | 20 | 8 | 30 | 10 | 8 |
| | Average output (W) | | 50 | 35 | 10 | 6 | 10 | 6 | 6 |
| | Pulse width (ns) of laser | | 150 | 100 | 50 | 20 | 10 | 7 | 5 |
| | Q-switch frequency | | 25 | 25 | 30 | 30 | 35 | 30 | 30 |
| | Scanning speed | | 2000 | 2000 | 2000 | 2000 | 2000 | 2000 | 2000 |
| Assessments | Contrast | 3 or more | 2 | 3 or more | 3 or more | 3 or more | 3 or more | 3 or more | 3 or more |
| | Visibility | | 4 | 2 | 1 | 1 | 3 | 1 | 1 |

Examples 1-6 illustrate the results of laser marking which was applied to the endoscope 10 which was formed of polyester resin employed as one example of thermoplastic resin. Almost the same effects were obtained even if the endoscope 10 was formed by making use of thermosetting resin or rubber.

The quantity of the colorants and the fillers of the resin composition shown in Table 1 is represented by weight parts based on 100 parts by weight of the polyester resin. In Examples 1-6 and Comparative Example, one weight part of carbon black, 0.1 weight part of calcium carbonate, 0.1 weight part of black iron oxide and one weight part of titanium dioxide were added as a colorant/filler based on 100 parts by weight of the thermoplastic polyester resin, and then the pulse laser beam 2 was irradiated to the thermoplastic polyester resin containing the colorant to obtain a white marking portion, the results being illustrated in these examples and comparative example. Specifically, these Examples 1-6 illustrate these results obtained as the irradiation conditions of pulse laser beam 2 were varied. Comparative Example illustrates the results obtained as the irradiation of pulse laser beam 2 was performed according to the prior art.

The irradiation conditions of pulse laser beam 2 include laser species (laser beam source 1), e.g., YAG laser or YVO₄ laser; wavelength; the intervals of hatching h; the spot diameter r; peak output; average output; pulse width; the Q-switching frequency Qf; and the scanning speed Sp.

Conventional Example illustrates the results obtained as a white marking was applied to a layer of black thermoplastic polyester resin by making use of a white thermosetting urethane ink.

This table shows the results of assessment with respect to contrast and visibility. The results of contrast shown therein was obtained from the measurement performed by making use of a luminance meter. The results of visibility shown therein was obtained from the visual assessment made in comparison with the printing using ink. With respect to the contrast, all of Examples 1-6 indicated "3" or more. Whereas, the contrast in the case of the conventional example was not less than "3", and the contrast in the case of the comparative example was "2".

The visibility was assessed according to four phases, e.g., "1" to "4" for instance. The phase "1" means a white color development which was comparable to ink, thus indicating very excellent visibility. The phase "2" means a white color development which was somewhat inferior to ink, but was still excellent in visibility. The phase "3" means a white color development which was inferior as compared with ink, thus indicating poor visibility. The phase "4" means that it was hardly possible to recognize color development, thus indicating poor visibility.

Table 1 also show the results of measurements with respect to brightness L*, shade a* and chroma b* which were measured by making use of an L*a*b* color specification meter, the procedure of which was specified in JIS Z8729 wherein the colors of Examples 1-6 were measured using a color spectrometer (Konica-Minolta Co., Ltd.). The larger the L* value is and the smaller the values of a* and b* are, the more excellent in whiteness. According to this measurement, the conventional ink was large in L* value, indicating excellent whiteness. In the cases of Examples 1-6, the L* value thereof was 60 or more, indicating excellent visibility.

The results of the assessment performed as described above indicate that as long as the irradiation conditions of pulse laser beam 2 are confined to those of Examples 1-6, it is possible to secure excellent contrast and visibility as seen in Examples 1-6. Thus, it was possible, according to Examples 1-6, to obtain excellent markings by means of laser marking, the quality of which was comparable to the printed matter created by making use of ink. Whereas, if the irradiation conditions of pulse laser beam are set according to the prior art (Comparative Example), the contrast would be degraded, resulting in poor visibility. It will be understood from these results that the present invention (Examples 1-6) is more excellent in contrast and visibility.

As described above, according to the aforementioned first embodiment, a colorant is incorporated in thermoplastic resin, thermosetting resin or rubber and then a pulse laser beam of YAG or YVO₄ having a wavelength of 355, 532 or 1064 nm is irradiated to a portion containing this colorant, thereby enabling this portion to color-develop, thus forming markings including letters and symbols. In this manner, it is possible to obtain the endoscope 10 which is provided, on the surface thereof, with markings 15-20 each having a contrast of "3" or more and being comparable in white color development to a printed matter to be created by making use of ink and hence very excellent in visibility.

The endoscope 10 is generally sterilized by means of a sterilization method using a glutaric aldehyde-based sterilizing liquid, a sterilizing liquid containing peracetic acid or a sterilizing liquid containing hydrogen peroxide; a sterilization method using hydrogen peroxide and low-temperature plasma; or an autoclave sterilization method. Even if these sterilization methods are applied to the endoscope 10, each of the markings 15-20 applied to the endoscope 10 may not be peeled off or discolored to such an extent to make them difficult to identify.

Although the endoscope 10 is frequently rubbed in the washing thereof, the markings 15-20 can be hardly rubbed away during such a washing.

According to the method of applying these markings 15-20 to the endoscope 10 by means of laser marking, an ink containing an organic solvent is no longer required to be employed. Because of this, this method would not give any adverse influence to environments and, moreover, it is possible, according to this method, to enhance the safety to working personnel.

According to the laser marking, it is possible to apply these markings 15-20 to the endoscope 10 at a high-velocity. Because of this, the endoscope 10 can be mounted on a high-speed endoscope-manufacturing line.

Each of the inserting flexible tube 11, the manipulation portion 12 and the connector portion 13 of the endoscope 10 for example is frequently contacted with an operator or with a body cavity on the occasion when the endoscope 10 is employed in the observation, examination, diagnosis or treatment of human body. However, even if these markings 15-20 are attached to these portions of the endoscope 10, there is little possibility that these markings 15-20 are discolored or rubbed away.

Accordingly, the marking portion 18 applied to the manipulation portion 12 for indicating various kinds of angles such as a UD angle knob, a UD angle-canceling knob, an RL angle knob and an RL angle-canceling knob, as well as the marking portion 19 applied to the manipulation portion 12 for indicating various kinds of buttons such as a suction button, an air/water feeding button can be clearly identified. As a result, the manipulability of endoscope 10 can be enhanced. Especially, each of the marking portions 15 and 16 applied to the inserting flexible tube 11 is caused to frequently contact with a body cavity as the inserting flexible tube 11 is inserted into a body cavity. Even if these marking portions 15 and 16 are contacted with the body cavity, there is little possibility that these marking portions 15 and 16 are discolored or become blurred. Therefore, white indicator lines such as distance marks can be clearly recognized, thus making it possible to accurately determine the length to be inserted into a body cavity.

By the way, the aforementioned first embodiment may be modified as follows.

Although the aforementioned first embodiment has been discussed about the case where laser marking is applied to the endoscope 10 formed of thermoplastic resin, thermosetting resin or rubber, the present invention is not limited to such a case. Namely, a marking can be clearly applied to a metallic material such as stainless steel, etc., for instance.

Although the aforementioned first embodiment has been discussed about the case where marking portions 15-20 are applied to the endoscope 10, the present invention is not limited to such a case. Namely, the present invention can be applied to treating tools such as a biopsy, a rotary clip device, a high-frequency snare, etc., which can be employed as a medical instrument for endoscope which is designed to be employed together with the endoscope 10, wherein a colorant is incorporated in thermoplastic resin, thermosetting resin or rubber and then a pulse laser beam of YAG or YVO₄ having a wavelength of 355, 532 or 1064 nm is irradiated to a portion containing this colorant, thereby enabling this portion to color-develop, thus forming markings including letters and symbols.

According to the aforementioned first embodiment, it is possible to manufacture an endoscope 10 and a medical instrument for endoscope such as a treating tool each provided with marking portions representing letters or marks including symbols, wherein a colorant is incorporated in thermoplastic resin, thermosetting resin or rubber constituting the endoscope 10 and the medical instrument for endoscope such as a treating tool and then a pulse laser beam of YAG or YVO₄ having a wavelength of 355, 532 or 1064 nm is irradiated to a portion containing this colorant, thereby enabling this portion to color-develop, thus forming the marking portions.

The endoscope and the medical instrument for endoscope 3 are at least partially formed of thermoplastic resin, thermosetting resin or rubber and are provided with a portion which is formed of thermoplastic resin, thermosetting resin or rubber each containing a colorant or a filler. Then, a pulse laser beam of YAG or YVO₄ having a wavelength of 266 nm is irradiated to this portion. As a result of this, it is possible to provide an endoscope and a medical instrument for endoscope 3 with marking portions representing letters or marks including symbols which have been developed through the irradiation of pulse laser beam to the aforementioned portion.

Next, a second embodiment of the present invention will be explained with reference to drawings. By the way, in these drawings, the same portions as those of FIG. 6 are identified by the same reference symbols, thus omitting the explanation thereof.

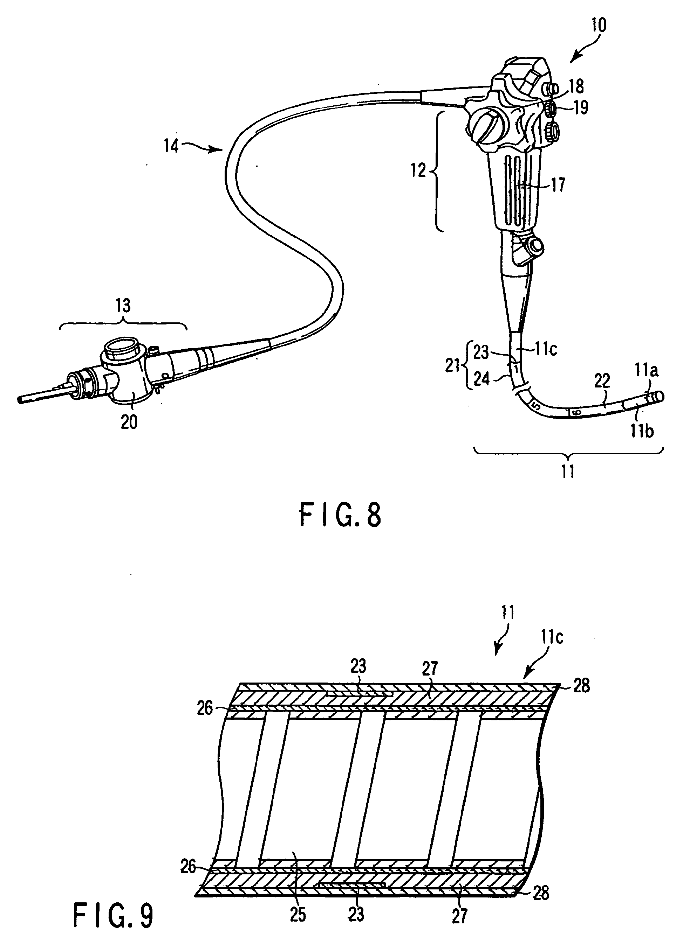

FIG. 8 shows an external appearance of the endoscope 10. The inserting flexible tube 11, manipulation portion 12 and connector portion 13 of the endoscope 10 are respectively provided with marking portions 21, 22, 17-19, 20 which can be applied thereto by making use of the laser marking apparatus shown in FIG. 1.

For example, the inserting flexible tube 11 is provided with the marking portions 21 and 22. The marking portion 21 shows a graduation (visible marker) 23 and the numerical values thereof 24. This graduation 23 is provided for measuring the depth of insertion of the inserting flexible tube 11 inserted into a body cavity. The numerical values 24 consist of "1, 2, 3, ..." for instance. These graduation 23 and numerical values 24 are respectively formed through the color development of a coloring agent as explained hereinafter. The graduation 23 is formed along the longitudinal direction of the inserting flexible tube 11 with the intervals (pitch) thereof being 10 cm and the width thereof being 1-10 cm for example. The intervals of the graduation 23 may be optionally selected. The marking portion 22 indicates a logotype.

The graduation 23 to be attached to the inserting flexible tube 11 will be explained with reference to the longitudinal sectional view of the inserting flexible tube 11 shown in FIG. 9. The inserting flexible tube 11 comprises a cylindrical spiral tube 25. On the outer circumferential surface of the spiral tube 25, there is laminated a mesh tube 26. On the outer circumferential surface of the mesh tube 26, there is laminated a skin layer 27. On the outer circumferential surface of the skin layer 27, there is laminated a cover layer 28. The skin layer 27 is provided, on the outer circumferential surface thereof, with the graduation 23 and the numerical values 24 indicating the depth of insertion (not shown in FIG. 2).

The skin layer 27 is formed of a material comprising a resinous material as a major material. The resinous material constituting the skin layer 27 contains carbon black as a colorant/color-developing agent/filler, which is capable of developing a color as it is irradiated with a pulse laser beam.

As for the resinous material constituting the skin layer 27, it is possible to employ any kind of materials as long as they are flexible. More specifically, there is not any particular limitation as for the kind of the resinous material. For example, it is possible to employ polyolefin such as polyethylene, polypropylene, ethylene-propylene copolymer and ethylene-vinyl acetate copolymer (EVA), cyclic polyolefin, modified polyolefin, poly(vinyl chloride), poly(vinylidene chloride), polystyrene, polyamide, polyamide, polyamide imide, polycarbonate, poly(4-methylpentene-1), ionomer, acrylic resin, polymethyl methacrylate, acrylonitrile-butadiene-styrene copolymer (ABS resin), acrylonitrile-styrene copolymer (AS resin), butadiene-styrene copolymer, polyoxymethylene, polyvinyl alcohol (PVA), ethylene-vinyl alcohol copolymer (EVOH), polyester such as polyethylene terephthalate (PET), polybutylene terephthalate (PBT) and polycyclohexane terephthalate (PCT), polyether, polyether ketone (PEK), polyetherether ketone (PEEK), polyether imide, polyacetal (POM), polyphenylene oxide, modified polyphenylene oxide, polysulfone, polyether sulfone, polyphenylene sulfide, polyallylate, aromatic polyester (liquid crystal polymer), polytetrafluoroethylene, polyvinylidene fluoride, other kinds of fluorinated resin, various kinds of thermoplastic elastomers such as polystyrene-based, polyolefin-based, polyvinyl chloride-based, polyurethane-based, polyester-based, polyamide-based, polybutadiene-based, transpolyisoprene-based, fluorine-containing rubber-based and chlorinated polyethylene-based elastomers, various kinds of thermosetting resins such as urea resin, melamine resin, xylene resin, polyester resin, epoxy resin, phenol resin, furan resin, polybutadiene resin and polyurethane resin, various kinds of rubber such as natural rubber (NR), isoprene rubber (IR), butadiene-based rubber such as butadiene rubber (BR, 1, 2-BR) and styrene-butadiene rubber (SBR), chloroprene rubber (CR), diene-based special rubber such as butadiene-acrylonitrile rubber, butyl rubber (IIR), ethylene-propylene rubber (EPM, EPDM), acrylic rubber (ACM, ANM), olefin-based rubber such as butyl halide rubber (X-IIR), urethane-based rubber such as urethane rubber (AU, EU), ether-based rubber such as hydrin rubber (CO, ECO, GCO, EGCO), polysulfide-based rubber such as polysulfide rubber (T), silicone rubber (Q), chlorinated polyethylene (CM), copolymers or blend polymers mainly comprising any of these various kinds of rubber, polymer alloy, etc. These resinous materials may be employed singly or in combination of two or more kinds thereof.

Among these resinous materials, it is especially preferable to employ thermoplastic elastomers such as polyurethane-based, polystyrene-based, polyester-based, polyolefin-based elastomers, polyethylene and polypropylene. These thermoplastic elastomers such as polyurethane-based, polystyrene-based, polyester-based, polyolefin-based elastomers, polyethylene and polypropylene are excellent in chemical resistance. Because of this, it is possible to enhance the durability of marking portions to repeated washing, disinfection and sterilization treatments that may be conducted to the endoscope 10.

An average thickness of the skin layer 27 should be sufficient enough to protect the inner materials disposed in the inserting flexible tube 11. As long as the flexibility and bendability of the inserting flexible tube 11 are not hindered, there is not any particular limitation with respect to the average thickness of the skin layer 27. Preferably however, the average thickness of the skin layer 27 should be confined to the range of, for example, 100-3000 µm or so. More preferably, the average thickness of the skin layer 27 should be confined to the range of 200-1000 µm or so.

As for the color-developing agent, carbon black is generally employed. If the particle diameter of carbon black is 100 nm or more, the dispersion of carbon black may become insufficient, thus more likely giving rise to non-uniformity in development of color. Therefore, the average particle diameter of carbon black should preferably be confined to the range of 10-80 nm. More preferably, the average particle diameter of carbon black should be confined to the range of 12-40 nm. Carbon black should preferably be incorporated into a resinous material constituting the skin layer 27 at a ratio ranging from 0.001 to 25 parts by weight, more preferably from 1 to 5 parts by weight.

As for the colorant, it is preferable to employ black iron oxide and titanium black. As for the colorant to be used for promoting the whiteness of markings, it is preferable to employ titanium dioxide and calcium carbonate.

As for the additives other than those mentioned above, it is possible to employ, for example, an inorganic filler, a lubricant, a plasticizer, various kinds of stabilizers (for example, an antioxidant, a light stabilizer, an antistatic agent, an anti-blocking agent), a release agent, a flame retardant, a coupling agent, an X-ray contrast medium, etc. As for specific examples of the lubricant, they include stearic acid, behenic acid or the esters or salts thereof, carnauba wax, polyethylene wax, various kinds of surfactants, etc.

Next, a method of forming the graduation (visible marker) 23 will be explained. Namely, a pulse laser beam is irradiated onto a predetermined portion of the outer surface of skin layer 27 where a color-developing agent is incorporated in advance, thereby enabling the color-developing agent to color-develop by the energy of pulse laser beam.

As for the pulse laser beam to be employed herein, it is possible to employ carbon dioxide laser, He-Ne laser, ruby laser, semiconductor laser, argon laser, excimer laser, YVO₄ laser, YAG laser, etc. These lasers are very powerful so that they are capable of fusing the surface of resinous material constituting the skin layer 27.

When laser marking is to be applied to thermoplastic resin, thermosetting resin or rubber, the wavelength of pulse laser beam 2 may be selected from any of 1064, 532 and 355 nm in executing the laser marking.

Therefore, Nd:YVO₄ laser (hereinafter referred to as YVO₄ laser) which is capable of outputting a pulse laser beam 2 having a wavelength of 1064 nm for example can be employed as the pulse laser beam source 1. Alternatively, Nd:YAG laser (hereinafter referred to as YAG laser) which is capable of outputting a pulse laser beam 2 having a wavelength of 1064 nm can be employed as the pulse laser beam source 1. These YAG laser and YVO₄ laser may be enabled to output a pulse laser beam 2 having a wavelength of 355 or 532 nm as they are subjected to a half-wave conversion.

When laser marking is to be applied to thermoplastic resin, thermosetting resin, rubber or thermoplastic elastomer, the wavelength of pulse laser beam 2 to be emitted from the pulse laser beam source 1 may be selected from any of 1064, 532 and 355 nm in executing the laser marking.

A colorant/color-developing agent/filler has a strong absorption band to the wavelength of visible region and ultraviolet region. Therefore, the wavelength of pulse laser beam 2 should preferably, be selected from 532 and 355 nm which are capable of applying laser marking onto the surface of thermoplastic resin, thermosetting resin, rubber or thermoplastic elastomer at a low laser output while giving no damage to the endoscope 10. Even if the wavelength of pulse laser beam 2 is subjected to a half-wave conversion to thereby convert a wavelength of 1064 nm into a quarter thereof and then the resultant pulse laser beam 2 having a wavelength of 266 nm is irradiated as described above, it is possible of obtain excellent markings.

Next, the method of laser marking by making use of a laser marking apparatus will be explained.

Marking conditions are set to the controller 8 from the presetting section 9. As for the marking conditions, they may be set, for example, such that the spot diameter r of pulse laser beam 2 is confined within the range of 5-100 µm, the intervals of hatching h of pulse laser beam 2 are confined within the range of 1-80 µm, the Q-switching frequency Qf of pulse laser beam 2 is confined within the range of 0.1-100 kHz, and the scanning speed Sp of pulse laser beam 2 is confined within the range of 1-3000 mm/sec.

More specifically, the spot diameter r of pulse laser beam 2 is set to not more than 40 µm, the intervals of hatching h of pulse laser beam 2 is set to 30 µm, the Q-switching frequency Qf of pulse laser beam 2 is set to 30 kHz, and the scanning speed Sp of pulse laser beam 2 is set to the range of about 2-3000 mm/sec.

By means of the controller 8, the switching operation of the Q-switch 1c, the scanning speed Sp of each of the X-axis scanner 5 and the Y-axis scanner 6 of XY scanner 4 and the Q-switching frequency Qf of the Q-switch 1c are respectively controlled according to the preset marking conditions including the intervals of hatching h of pulse laser beam 2, the Q-switching frequency Qf of pulse laser beam 2 and the scanning speed Sp of pulse laser beam 2.

By doing so, the pulse laser beam 2 emitted from the pulse laser beam source 1 is scanned in the Y-axis direction by means of the Y-axis scanner 6 and also scanned in the X-axis direction by means of the X-axis scanner 5 before it is transmitted to the fθ lens 7. The pulse laser beam 2 transmitted to the fθ lens 7 is then scanned as a spot light over the surfaces of endoscope 10. The spot diameter r of pulse laser beam 2 at this time is converged by means of this fθ lens 7 to a diameter of 5-100 µm, especially to not more than 40 µm.

As the pulse laser beam 2 is irradiated onto the surface of the skin layer 27 where the graduation 23 of the endoscope 10 is to be created, the skin layer 27 that has been irradiated with the pulse laser beam 2 is discolored into whitish color. The pulse laser beam 2 is scanned in the XY-axes every pulse. As a result, a portion of the skin layer 27 that has been irradiated with the pulse laser beam 2 during this scanning is discolored into whitish color.

Namely, the color of the outer surface of skin layer 27 is black before this outer surface is irradiated with the pulse laser beam 2. However, when the outer surface of skin layer 27 is irradiated with the pulse laser beam 2, the pulse laser beam 2 is absorbed by the carbon black contained in the skin layer 27, thus allowing carbon existing in this irradiated portion to vaporize. The black component at this stage is caused to vanish or reduce.

The resinous component of skin layer 27 is capable of absorbing the pulse laser beam 2 and converting the pulse laser beam 2 into heat. The heat thus generated is acted on the macromolecule of the thermoplastic resin to decompose this macromolecule, thus expanding the irradiated portion. As a result, since the expanded portion differs in refractive index from the portion which is not irradiated with the pulse laser beam 2, the expanded portion would be no longer black. Namely, a portion of the surface of skin layer 27 which is irradiated with the pulse laser beam 2 is now caused to indicate irregular reflection, thus turning it white in color. As a result of this, the graduation 23 is formed on the surface of endoscope 10.

The inserting flexible tube 11, manipulation portion 12 and connector portion 13 of the endoscope 10 are respectively provided with marking portions 22, 17-19, 20 which can be applied thereto by means of laser marking in addition to the graduation 23 and the numerical values 24. Namely, the pulse laser beam 2 is irradiated onto portions of the skin layer 27 of the endoscope 10 where these marking portions 22, 17-19, 20 are respectively located, thus discoloring these portions of the skin layer 27 into whitish surfaces.

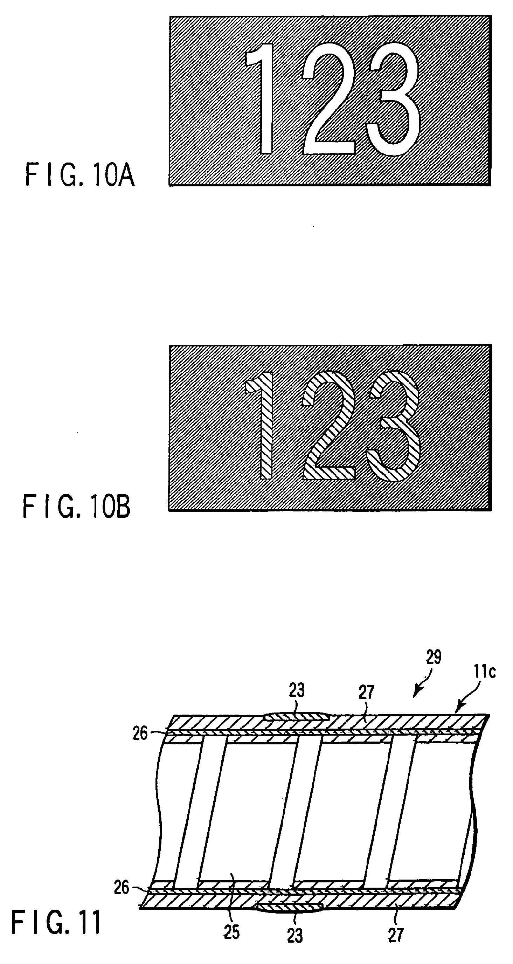

FIGS. 10A and 10B illustrate the comparison between the present invention and the prior art. Namely, FIG. 10A shows one example of the numerical values 24 of marking portion 21 applied to the endoscope 10 of the present invention. FIG. 10B shows a marking portion which was applied to the endoscope 10 by means of the conventional laser marking. It will be recognized from the comparison of these marking portions that the marking portion 21 applied to the endoscope 10 of the present invention was more excellent in contrast and visibility as compared with the conventional marking portion.

Next, Examples 1-3, an example according to the prior art, and Comparative Example will be explained with reference to Table 2.

**Table 2**

| | Conventional example 1 | Comparative example | Example 1 | Example 2 | Example 3 |
|---|---|---|---|---|---|
| Marking method | Ink | Laser | Laser | Laser | Laser |
| Resin for flexible tube | Black thermoplastic elastomer | Black Thermoplastic elastomer | Black thermoplastic elastomer | Black thermoplastic elastomer | Black Thermoplastic elastomer |
| Laser species | | YAG | YAG | YV0₄ | YV0₄ |
| Wavelength (nm) | | 1064 | 1064 | 532 | 355 |
| Laser peak output | | 100 | 100 | 20 | 8 |
| Laser spot diameter | | 100 | 100 | 40 | 30 |
| | | | | | |
| Assessment Test A | ○ | × | ○ | ○ | ○ |
| Assessment Test B | ○ | × | ○ | ○ | ○ |
| Assessment Test C | ○ | × | ○ | ○ | ○ |

In each of Examples 1-3 and Comparative Example, these marking portions 22, 17-19, 20 were respectively formed by making use of laser marking wherein a pulse laser beam was irradiated thereto from YAG laser and YVO₄ laser. In the Conventional Example, the markings were created by making use of ink.

In each of Examples 1-3, Comparative Example and Conventional Example, a black thermoplastic elastomer was employed as a resin for the inserting flexible tube 11.

In each of Examples 1-3, the Wavelength of the pulse laser beam 2 employed therein was 1064, 532 and 355 nm, respectively. In Comparative Example, the wavelength of the pulse laser beam 2 employed therein was 1064 nm.

In each of Examples 1-3, the peak output of pulse laser beam 2 employed therein was 100 "unit", 20 "unit" and 8 "unit", respectively. In Comparative Example, the peak output of pulse laser beam 2 employed therein was 100 "unit".

In each of Examples 1-3, the spot diameter r of the pulse laser beam 2 employed therein was 100 µm, 40 µm and 30 µm, respectively. In Comparative Example, the spot diameter r of the pulse laser beam 2 employed therein was 100 µm.

Next, the average particle diameter and composition of the carbon employed in each of Examples 1-3 will be explained.

In Example 1, the composition of resin employed for the flexible tube was formed of 100 parts by weight of polyolefin elastomer, 10 parts by weight of carbon black having an average particle diameter of 20 nm, 20 parts by weight of calcium carbonate having an average particle diameter of 5 nm, 0.1 part by weight of black iron oxide having an average particle diameter of 0.5 µm, and 0.1 part by weight of titanium black having an average particle diameter of 0.1 µm.

In Example 2, the composition of resin employed for the flexible tube was formed of 100 parts by weight of polyolefin elastomer, 3 parts by weight of carbon black having an average particle diameter of 20 nm, 20 parts by weight of calcium carbonate having an average particle diameter of 5 nm, one part by weight of black iron oxide having an average particle diameter of 0.5 µm, and 0.1 part by weight of titanium black having an average particle diameter of 0.1 µm.

In Example 3, the composition of resin employed for the flexible tube was formed of 100 parts by weight of polyolefin elastomer, one part by weight of carbon black having an average particle diameter of 20 nm, and 30 parts by weight of calcium carbonate having an average particle diameter of 7 nm.

In Conventional Example 1, a core material was manufactured in the same manner as in Example 1. Then, a skin layer (0.4 nm in average thickness) formed of polyurethane-based thermoplastic elastomer (Bandex; DIC Bayer Polymer Co., Ltd.) containing no color-developing agent was coated on the outer circumference of the core material by means of extrusion molding.

Then, a polyurethane coating material (ink) was printed on the outer circumference of the skin layer, thereby obtaining the inserting flexible tube.

Then, by making use of this inserting flexible tube, an endoscope (an endoscope for upper alimentary canal) as shown in FIG. 8 was manufactured.

In Comparative Example 1, a core material was manufactured in the same manner as in Example 1. Then, a skin layer 22 (0.4 nm in average thickness) formed of polyurethane-based thermoplastic elastomer (Bandex; DIC Bayer Polymer Co., Ltd.) containing no color-developing agent was coated on the outer circumference of the core material by means of extrusion molding.

Then, by way of laser working using YAG laser and YVO₄ laser, projected/recessed portions representing a graduation were formed on the outer surface of skin layer. In this manner, an inserting flexible tube was obtained. Then, by making use of this inserting flexible tube, an endoscope (an endoscope for upper alimentary canal) 10 as shown in FIG. 8 was manufactured.

Next, there will be explained about the assessment tests "A", "B" and "C" which were performed on each of endoscopes 10 manufactured in Examples 1-3, Comparative Example and Conventional Example.

In the assessment test "A" (assessment of whiteness), the whiteness (L-value) of the graduation 23 of each of the endoscopes 10 that had been manufactured in Examples 1-3 and Comparative Example was measured by making use of a whiteness meter (NW-1; Nippon Denshoku Industries Co., Ltd.), the results being assessed according to the following two phases of criterion. In Table 2, "O" means 60% or more and "X" means less than 60%.

In the assessment test "B" (assessment of visibility), the graduation 23 of each of the endoscopes 10 that had been manufactured in Examples 1-3, Comparative Example and Conventional Example was visually observed, the visibility being assessed according to the following four phases of criterion. In Table 2, "☆" means very excellent, "O" means excellent, "Δ" means somewhat poor, and "X" means poor.

In the assessment test "C", each of the endoscopes 10 that had been manufactured in Examples 1-3 and Comparative Example was subjected to 300 cycles of disinfection (conditions: pH=2.5±0.2; redox potential=1100 mV; effective chlorine concentration=50 ppm) by making use of a sterilizing apparatus using strong acidic water. After finishing the 300 cycles of disinfection, the region of graduation was visually observed, the visibility being assessed according to the following four phases of criterion.

In Table 2, "☆" means that the graduation 23 was retained in a clear state and no deterioration of skin layer 27 was recognized at all, "O" means that the graduation 23 became somewhat unclear but the deterioration of skin layer 27 was not recognized, "Δ" means that the graduation 23 became unclear and the skin layer 27 in the vicinity of the graduation 23 was roughened, and "X" means that the graduation 23 could not be recognized and the skin layer 27 in the vicinity of the graduation 23 was remarkably roughened.

As seen from the results of Examples 1-3 shown in Table 1, the laser markings of the present invention all exhibited excellent properties. The endoscopes 10 that had been manufactured in Examples 1-3 were all high in whiteness (L-value) of the graduation 23 and also very excellent in visibility. Further, the endoscopes 10 that had been manufactured in Examples 1-3 all retained a clear state of graduation 23 even after 300 cycles of disinfection in the assessment test "C".

Whereas, the endoscope that had been manufactured in Comparative Example was low in whiteness (L-value) of the graduation 23 and also very poor in visibility. Further, when the graduation 23 of the endoscope was wiped with ethanol after 300 cycles of disinfection in the assessment test "C", the graduation 23 was permitted to peel away.

As described above, according to the aforementioned second embodiment, 0.001-20 parts by weight of carbon black having an average particle diameter ranging from 10 to 80 nm is incorporated in thermoplastic elastomer and then a pulse laser beam of YAG or YVO₄ having a wavelength of 355, 532 or 1064 nm is irradiated to a portion containing this carbon black, thereby enabling this portion added with carbon black to color-develop, thus forming the marking portion 21 consisting of the graduation 23 and the numerical values 24 and also forming the markings 22, 17-19, 20 representing the identification name or logotype of the endoscope 10 or buttons. In this manner, it is possible, by means of laser marking, to provide the endoscope 10 with markings 21, 22, 17-19, 20 each having excellent contrast and visibility which are comparable in quality to a printed matter to be created by making use of ink.

Since the graduation 23 can be formed through the color development of a color-developing agent which can be effected by the irradiation of pulse laser beam 2, the graduation 23 can be hardly peeled away, vanished or faded away.

If the graduation 23 is to be formed on the outer surface of skin layer 27 by means of printing, a step for drying the ink will be required. Whereas, in the case of laser marking as proposed by the present invention, the drying step of ink is no longer required and therefore, the present invention is advantageous in that the formation of graduation 23 can be accomplished within a short period of time.

By the way, the aforementioned second embodiment can be modified as follows.

FIG. 11 shows a longitudinal sectional view of an inserting flexible tube 29 of the endoscope 10. This inserting flexible tube 29 differs from the inserting flexible tube 11 explained with reference to the aforementioned second embodiment in the respect that the covering layer 28 is deleted from the inserting flexible tube 11. The skin layer 27 of the inserting flexible tube 29 is provided, on the outer circumferential surface thereof, with the graduation 23 and the numerical values 24 (not shown in FIG. 11) representing the depth of insertion.

As in the case of the aforementioned second embodiment, the graduation 23 and the numerical values 24 can be formed by a process wherein 0.001-20 parts by weight of carbon black having an average article diameter ranging from 10 to 80 nm is incorporated in thermoplastic elastomer and then a pulse laser beam of YAG or YVO₄ having a wavelength of 355, 532 or 1064 nm is irradiated to a portion containing this carbon black, thereby enabling this portion added with carbon black to color-develop, thus forming the graduation 23 and the numerical values 24. By the way, the endoscope 10 may be provided with the marking portions 22, 17-19, 20 representing the identification name or logotype of the endoscope 10 or buttons as in the case of the aforementioned second embodiment in addition to the marking portion 21 consisting of the graduation 23 and the numerical values 24.

Even in the cases of the marking portions 22, 17-19, 20 which are attached to the inserting flexible tube 29 of the endoscope 10, it is of course possible to obtain almost the same effects as obtainable in the aforementioned second embodiment.

Although the skin layer 27 is formed of a single layer in the aforementioned second embodiment, the structure of the skin layer 27 should not be construed as limited to such a structure. For example, the skin layer 27 may be constructed such that the skin layer 27 is partially or entirely (i.e., in a circumferential direction) constituted by a laminate layer comprising a plurality of layers. In this case, it is only required that at least outmost layer thereof is formed of the same composition as the composition of the skin layer 27 employed in the aforementioned second embodiment.

Although the laser marking is applied to the endoscope 10 which is formed of thermoplastic resin, thermosetting resin, rubber or thermoplastic elastomer in the explanation of the aforementioned second embodiment, the present invention should not be construed as limited to such a case. For example, clear markings can be applied also to a metallic material such as stainless steel for instance.

Although the aforementioned second embodiment has been discussed about the case where marking portions 21, 22, 17-19, 20 are applied to the endoscope 10, the present invention is not limited to such a case. Namely, the present invention can be applied to treating tools such as a biopsy, a rotary clip device, a high-frequency snare, etc., which can be employed as a medical instrument for endoscope which is designed to be employed together with the endoscope 10, wherein 0.001-20 parts by weight of carbon black having an average particle diameter ranging from 10 to 80 nm is incorporated as a colorant/color-developing agent/filler in thermoplastic resin, thermosetting resin, rubber or thermoplastic elastomer and then a pulse laser beam of YAG or YVO₄ having a wavelength of 355, 532 or 1064 nm is irradiated to a portion containing this carbon black, thereby enabling this portion to color-develop, thus forming markings including letters and symbols.

According to the present invention, it is possible to manufacture an endoscope 10 and a medical instrument for endoscope such as a treating tool each provided with marking portions 21, 22, 17-19, 20 representing letters or marks including symbols, wherein 0.001-20 parts by weight of carbon black having an average particle diameter ranging from 10 to 80 nm is incorporated as a colorant/color-developing agent/filler in thermoplastic resin, thermosetting resin or rubber constituting the endoscope 10 and the medical instrument for endoscope such as a treating tool and then a pulse laser beam of YAG or YVO₄ having a wavelength of 355, 532 or 1064 nm is irradiated to a portion containing this carbon black, thereby enabling this portion to color-develop, thus forming these marking portions.

Next, a third embodiment of the present invention will be explained with reference to drawings. By the way, in these drawings, the same portions as those of FIG. 6 are identified by the same reference symbols, thus omitting the explanation thereof.

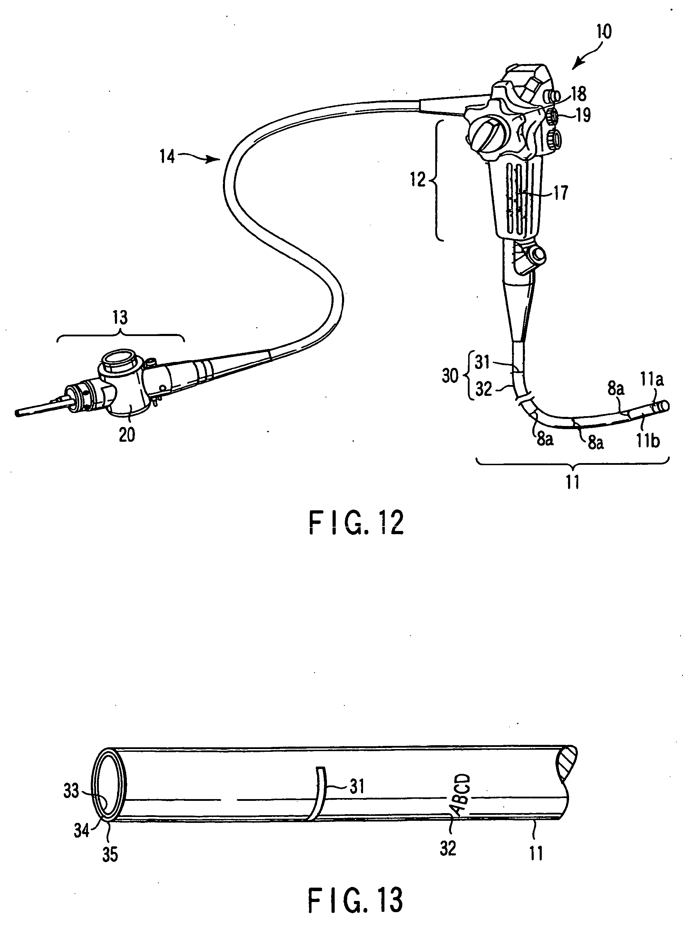

FIG. 12 shows an external appearance of the endoscope 10. The inserting flexible tube 11, manipulation portion 12 and connector portion 13 of the endoscope 10 are respectively provided with marking portions 30, 17-20 which are formed by means of the laser marking. The laser markings are formed by irradiating laser beam to each of the inserting flexible tube 11, the manipulation portion 12 and the connector portion 13, or by irradiating laser beam to a portion of each of the inserting flexible tube 11, the manipulation portion 12 and the connector portion 13 where a colorant/color-developing agent/filler has been incorporated therein in advance, thereby enabling the color-developing agent to develop the color thereof.

The inserting flexible tube 11 is provided with the marking portion 30. In this marking portion 30, there are formed a plurality of indicator lines (graduation and visible marker) 31 as a first indicator for measuring the depth of insertion of the inserting flexible tube 11 into a body cavity and the name (logotype) of manufacturer of the endoscope as a second indicator.

The plurality of indicator lines 31 are respectively formed at predetermined intervals along the axial direction of the inserting flexible tube 11. The intervals of each of indicator lines 31 may be optionally selected. The numerical values such, for example, as "1, 2, 3, ..." indicating the depth of insertion may be formed in the inserting flexible tube 11 together with and along the indicator lines 31.

FIG. 13 shows an external appearance of the inserting flexible tube 11 having formed thereon the indicator lines 31 and the name (logotype) 32 of manufacturer. The inserting flexible tube 11 comprises a cylindrical spiral tube 33. The outer circumferential surface of this spiral tube 33 is covered with a mesh tube 34. The outer circumferential surface of this mesh tube 34 is covered with a flexible tube 35 as a skin layer. Namely, the inserting flexible tube 11 is formed of a laminate consisting of the spiral tube 33, the mesh tube 34 and the flexible tube 35 which are concentrically laminated. In the interior of the spiral tube 33, there are inserted various kinds of inner materials such as a fiber bundle, a tube, etc. In this manner, the inserting flexible tube 11 is formed into a long and slender tube having a circular cross-section.

On the outer circumferential surface of the inserting flexible tube 11, there are attached a plurality of indicator lines 31 and the name (logotype) 32 of manufacturer such as "ABCD". The plurality of indicator lines 31 are formed, starting from the distal end portion 11a of the inserting flexible tube 11 for example, at intervals (pitch) of 10 cm for example and along the direction perpendicular to the axial direction of the inserting flexible tube 11, i.e., in the circumferential direction along the outer circumferential surface of the inserting flexible tube 11 the width thereof being set to 1-10 mm.

The name (logotype) 32 of manufacturer is formed at a proximal end of the inserting flexible tube 11 in the same direction as the direction of the indicator lines 31, i.e., in the circumferential direction along the outer circumferential surface of the inserting flexible tube 11.

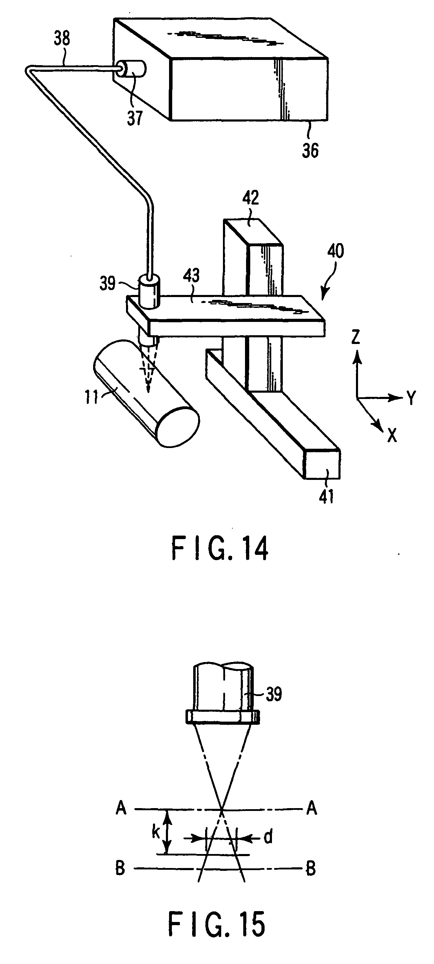

FIG. 14 illustrates the construction of a laser marking apparatus for forming the marking portions 30, 17-20, for example, at the inserting flexible tube 11, manipulation portion 12 and connector portion 13 of the endoscope 10, respectively. The laser beam source 36 is designed to emit a pulse laser beam by making use of, for example, YAG laser or YVO₄ laser.

The laser beam source 36 is connected, through the outgoing end thereof, with one end of an optical fiber 38. The other end of the optical fiber 38 is connected with a converging optical system 39. This optical fiber 38 has a core having a diameter of several hundreds micrometers for example.

The converging optical system 39 is designed to converge the laser beam emitted through the optical fiber 38 from the laser beam source 36 to thereby create a spot beam at the outer circumferential surface of the inserting flexible tube 11. The optical magnification of the converging optical system 39 may be 0.5-2 times or so for example. The diameter of the core of optical fiber 38 may be several hundreds micrometers as described above. As a result, the diameter of beam waist of the laser beam thus converged by the converging optical system 39 may become 1 mm or so.

The diameter of beam of the laser beam can be expanded or shrunk by defocusing the converging optical system 39. By doing so, the diameter of beam of the laser beam can be optimized in conformity with the size of various kinds of markings at each of the marking portions 30, 17-20.

The converging optical system 39 is capable of minimizing the beam diameter d of laser beam in the state of just focusing as shown in FIG. 15. According to this converging optical system 39, the beam diameter d of the laser beam can be increased as the magnitude of defocusing is increased from the position of just focusing. Concomitant with this, the peak value in beam intensity of laser beam would be decreased. Even if the beam diameter is varied due to the defocusing, the intensity distribution of laser beam would always become such that is close to Gauss distribution. The line A-A in FIG. 15 indicates the position of just focusing of the converging optical system 39. The beam diameter k of laser beam represents the beam diameter at a position spaced away by a distance k from the position of just focusing.

A robot 40 is employed to actuate the converging optical system 39. By making use of this robot 40, the laser beam to be converged by the converging optical system 39 is moved to a designated portion of the inserting flexible tube 2. For example, if it is desired to perform the laser marking of the indicator lines 31 and the name (logotype) 32 of manufacturer, the position of irradiating the laser beam is caused to move by means of the robot 40 over the surface of flexible tube 35 of the inserting flexible tube 11 according to the specific configuration of the indicator lines 31 and of the name (logotype) 32 of manufacturer.

The robot 40 is constituted by a frame 41 mounted movable in the X-axis direction, a post 42 mounted on the frame 41 and enabled to move in the Y-axis direction, and a supporting arm 43 attached to the post 42 and enabled to move in the Y-axis direction as well as in the Z-axis direction. The converging optical system 39 is mounted on a distal end portion of the supporting arm 437.

Next, there will be explained a method of forming the marking portion 30 on the surface of endoscope 10 wherein the laser marking apparatus constructed as described above is employed.

The inserting flexible tube 11 is disposed enabling it move in the X-axis direction which is parallel with the frame 41.

The laser beam is emitted from the laser beam source 36. The laser beam is transmitted through the optical fiber 38 and introduced into the converging optical system 39. By means of this converging optical system 39, the laser beam transmitted through the optical fiber 38 is converged at the outer circumferential surface of the inserting flexible tube 11.

Under this condition, the post 42 is moved over the frame 41 and along the direction of X-axis by means of the robot 40 so that the position of irradiating the laser beam is caused to move over the surface of flexible tube 35 of the inserting flexible tube 11 according to the specific configuration of the indicator lines 31 and of the name (logotype) 32 of manufacturer. At the same time, by means of the robot 40, the supporting arm 43 is moved relative to the post 42 and in the direction of Y-axis. In this manner, the laser beam to be converged by means of the converging optical system 39 is irradiated to a designated portion of the inserting flexible tube 11.

Thus, on the occasion of forming the indicator lines 31 by means of laser marking, the supporting arm 43 is moved in the direction of Y-axis by making use of the robot 40. By doing so, the position of irradiating laser beam to the surface of flexible tube 35 on which the laser beam is to be converged by means of the converging optical system 39 is moved in the circumferential direction along the outer circumferential surface of the flexible tube 35 by making use of the robot 40.

On the occasion of forming the name (logotype) 32 of manufacturer by means of laser marking, the post 42 is moved in the direction of Y-axis by making use of the robot 40. At the same time, the supporting arm 43 is moved in the direction of Y-axis by making use of the robot 40. By doing so, the position of irradiating laser beam to be converged by means of the converging optical system 39 is moved in conformity with the configuration of the name (logotype) 32 of manufacturer such, for example, as "ABCD" by making use of the robot 40. In this case, the direction of forming the name (logotype) 32 of manufacturer such, for example, as "ABCD" would be also in the circumferential direction along the outer circumferential surface of the flexible tube 35.

As a result, there will be provided the inserting flexible tube portion 11 wherein the indicator lines 31 and the name (logotype) 32 of manufacturer are formed in the circumferential direction along the outer circumferential surface of the flexible tube 35 by means of laser marking.

The manipulation portion 12 is provided with a marking portion 17 indicating the type and name or logotype of the endoscopes 10, with a marking portion 18 indicating various kinds of angles such as a UD angle knob, a UD angle-canceling knob, an RL angle knob and an RL angle-canceling knob, and with a marking portion 19 indicating various kinds of buttons such as a suction button, an air/water feeding button, all being formed by means of laser marking. In the same manner as described above, the connector portion 13 is also provided with a marking portion 20 indicating the name of manufacturer or logotype for example, all being formed by means of laser marking.

As described above, according to the aforementioned third embodiment, the indicator lines 31 for determining the length of the inserting flexible tube portion 11 inserted into a body cavity as well as the name (logotype) 32 of manufacturer can be formed in the circumferential direction along the outer circumferential surface of the flexible tube 35 of endoscope 10.

In this manner, not only the indicator lines 31 for determining the length of the inserting flexible tube portion 11 inserted into a body cavity but also the name (logotype) 32 of manufacturer can be formed in the circumferential direction along the outer circumferential surface of the flexible tube 35. Therefore, it is possible to easily recognized not only the indicator lines 31 but also the name (logotype) 32 of manufacturer from the same visual direction.

By the way, the aforementioned third embodiment can be modified as follows.

The inserting flexible tube portion 11 may not be limited to one which is provided with the indicator lines 31 for determining the length of the inserting flexible tube portion 11 inserted into a body cavity and with the name (logotype) 32 of manufacturer. Namely, the inserting flexible tube portion 11 may be provided with the indicator lines 31 and with at least one kind of marking selected from markings including numerical values, letters and symbols such for example as the name of the manufacturer or dealer of endoscope 10, the manufacturer's serial number of endoscope 10 and the product name of endoscope 10.

The name (logotype) 32 of manufacturer, and the markings including numerical values, letters and symbols such for example as the name of the manufacturer or dealer of endoscope 10, the manufacturer's serial number of endoscope 10 and the product name of endoscope 10 may be formed by irradiating laser beam through a mask to the inserting flexible tube 11. By the way, the mask is provided with a light-transmitting portion corresponding in configuration to the name (logotype) 32 of manufacturer, and to the markings including numerical values, letters and symbols such for example as the name of the manufacturer or dealer of endoscope 10, the manufacturer's serial number of endoscope 10 and the product name of endoscope 10.

The movement of the irradiating position of laser beam may not necessarily performed by means of the robot 40. Namely, the movement of the irradiating position of laser beam may be performed in such a manner that the irradiating position of laser beam is fixed in place and the inserting flexible tube portion 11 is rotated about the axis thereof or moved linearly along the axial direction thereof.

The movement of the irradiating position of laser beam relative to the inserting flexible tube portion 11 may be performed in such a manner that the laser beam emitted from the laser beam source 36 is scanned by making use of a rotatable mirror and then the laser beam thus scanned is irradiated onto the inserting flexible tube portion 11. In this case, the scanning direction of laser beam is controlled by the rotational driving of mirror in conformity with the configuration of the indicator lines 31 or of the name (logotype) 32 of manufacturer.

By making of the laser marking, the indicator lines 31 or of the name (logotype) 32 of manufacturer may be applied not only to the endoscope 10 but also to treating tools to be utilized together with the endoscope 10 or to treating tools which can be utilized individually. To these treating tools may be attached the indicator lines 31 as well as at least one kind of marking selected from markings including numerical values, letters and symbols such for example as the name of the manufacturer or dealer of treating tools, the manufacturer's serial number of treating tools and the product name of treating tools.

For example, on the occasion of diagnosing or treating pancreatobiliary duct, a plurality of treating tools may be used in combination with each other. Namely, a treating tool such as a catheter for endoscope is inserted into a tool-inserting channel provided in the inserting flexible tube portion 11 of endoscope 10 and, at the same time, another kind of tool such as a guide wire is inserted into this catheter, thus enabling these tools to be utilized as a treating apparatus for endoscope.

On the occasion of using the treating apparatus, the inserting flexible tube portion 11 of endoscope 10 is inserted in advance into a cavity of patient such, for example, as duodenum. Then, a catheter and a guide wire (i.e., treating tools) are introduced through the tool-inserting channel of endoscope 10 that has been inserted into a body cavity, thus enabling these tools to reach an aimed region to perform the treatment or diagnosis. On this occasion, the provision of distance marker is required for determining the length inserted of the catheter. Additionally, the provision of distance marker is required for determining the protruded length of guide wire through the monitoring thereof by means of the endoscope 10.

The outer diameter of the catheter or of the guide wire is very small. However, the markings regarding the outer diameter as well as the product name of these catheter and guide wire can be formed thereon together with the distance marker.

In the formation of these distance marker, outer diameter and product name on the surface of catheter or of guide wire, laser beam is irradiated onto the skin layer of each of these catheter and guide wire while scanning the irradiating position of laser beam in conformity with the specific configuration of distance marker, outer diameter and product name, thereby enabling the skin layer to color-develop, thus forming the marking portions. In this case, the distance marker, the outer diameter or the product name is formed in a direction perpendicular to the axial direction of the catheter or the guide wire, i.e., in the circumferential direction along the outer circumferential surface thereof.

As a result, the distance marker, outer diameter or product name of the catheter or of the guide wire can be easily recognized from the same visual direction.

8y the way, the markings to be applied to the catheter and the guide wire may not be limited to distance marker, outer diameter or product name. Namely, the catheter and the guide wire may be provided with a distance marker and with at least one kind of marking selected from markings including numerical values, letters and symbols such for example as the name of the manufacturer or dealer of catheter or guide wire, the manufacturer's serial number of catheter or guide wire and the product name of catheter or guide wire.

## Claims

1. An endoscope and a medical instrument for an endoscope (3), wherein
the endoscope and the medical instrument are at least partially formed of thermoplastic resin, thermosetting resin, rubber or thermoplastic elastomer and are provided with a portion which is formed of thermoplastic resin, thermosetting resin, rubber or thermoplastic elastomer each containing 0.001 to 20 parts by weight of carbon black having an average particle diameter of 10-80 nm as a colorant/color-developing agent/filler; and that:
the endoscope and the medical instrument are provided with a marking portion (21) indicating marks including letters and symbols which are developed through an irradiation of the portion containing the carbon black with a pulse laser beam (2) of YAG or YVO₄ having a wavelength of 355, 532 or 1064 nm.

2. The endoscope and the medical instrument for endoscope according to claim 1, **characterized in that**, preferably, the carbon black has an average particle diameter of 12-40 nm.

3. The endoscope and the medical instrument for endoscope according to claim 1, **characterized in that** the marking portion (21) comprises thermoplastic resin, thermosetting resin, rubber or thermoplastic elastomer in which at least one kind of material selected from the group consisting of carbon black, calcium carbonate, black iron oxide, titanium black and titanium dioxide is incorporated as a colorant/color-developing agent/filler.

4. The endoscope and the medical instrument for endoscope according to claim 1, **characterized in that** the marking portion comprises a polyolefin-based elastomer blend which is obtained through the vulcanization of only an EPDM rubber phase out of a fused PP/EPDM formed of a blend of the thermoplastic resin and the rubber.

5. The endoscope and the medical instrument for endoscope according to claim 1, **characterized in that** a main body of the endoscope and the medical instrument for endoscope comprises an insertion portion (11) to be inserted into a subject, a manipulation portion (12) for manipulating the insertion portion (11), and a connector portion (13) to be connected at least with a signal processor (8) for processing an image of the subject; and
the marking portion (21) is formed at each of the insertion portion (11), the manipulation portion (12) and the connector portion (13).

6. The endoscope and the medical instrument for endoscope according to claim 5, **characterized in that** the marking portion (21) is formed on a skin (27) of each of the insertion portion (11), the manipulation portion (12) and the connector portion (13).

7. The endoscope and the medical instrument for endoscope according to claim 4, **characterized in that** the marking portion (21) comprises the thermoplastic resin, the thermosetting resin, the rubber or the thermoplastic elastomer including the polyolefin-based elastomer blend, in which 0.1-30 parts by weight of calcium carbonate having an average particle diameter ranging from 5 to 10 nm is incorporated.

8. The endoscope and the medical instrument for endoscope according to claim 4, **characterized in that** the marking portion (21) comprises the thermoplastic resin, the thermosetting resin, the rubber or the thermoplastic elastomer including the polyolefin-based elastomer blend, in which 0.1-5 parts by weight of black iron oxide having an average particle diameter ranging from 0.3 to 0.8 µm is incorporated.

9. The endoscope and the medical instrument for endoscope according to claim 4, **characterized in that** the marking portion (21) comprises the thermoplastic resin, the thermosetting resin, the rubber or the thermoplastic elastomer including the polyolefin-based elastomer blend, in which 0.1-5 parts by weight of titanium black or titanium dioxide having an average particle diameter ranging from 0.1 to 0.8 µm is incorporated.

10. A method of applying markings to an endoscope and a medical instrument for an endoscope (3), wherein
the endoscope and the medical instrument are at least partially formed of thermoplastic resin, thermosetting resin, rubber or thermoplastic elastomer and are provided with a portion which is formed of the thermoplastic resin, the thermosetting resin, the rubber or the thermoplastic elastomer in which 0.001-20 parts by weight of carbon black having an average particle diameter of 10-80 nm is incorporated as a colorant/color-developing agent/filler;
the portion incorporated with the carbon black is irradiated with a pulse laser beam (2) of YAG or YVO₄ having a wavelength of 355, 532 or 1064 nm, thereby developing a marking portion (21) indicating marks including letters and symbols.

11. The method according to claim 10,
**characterized in that** the carbon black having an average particle diameter, preferably, ranging from 12 to 40 nm is incorporated into the thermoplastic resin, the thermosetting resin, the rubber or the thermoplastic elastomer.

12. The method according to claim 10, **characterized in that** at least one kind of material selected from the group consisting of carbon black, calcium carbonate, black iron oxide, titanium black and titanium dioxide is incorporated, as a colorant/color-developing agent/filler, in the thermoplastic resin, the thermosetting resin, the rubber or the thermoplastic elastomer.

13. The method according to claim 10, **characterized in that** a polyolefin-based elastomer blend which is obtained through the vulcanization of only an EPDM rubber phase out of a fused PP/EPDM formed of a blend of the thermoplastic resin and the rubber is incorporated into the portion to be color-developed.

14. The method according to claim 10, **characterized in that** a main body of the endoscope and the medical instrument for endoscope comprises an insertion portion (11) to be inserted into a subject, a manipulation portion (12) for manipulating the insertion portion, and a connector portion (13) to be connected at least with a signal processor (8) for processing an image of the subject; and wherein
the marking including the letters and the symbols is formed at each of the insertion portion (11), the manipulation portion (12) and the connector portion (13) of the main body of the endoscope.

15. The method according to claim 14, **characterized in that** the marking including the letters and the symbols is formed at a skin of each of the insertion portion, the manipulation portion and the connector portion of the main body of the endoscope.

16. The method according to claim 13, **characterized in that** the marking portion comprises the thermoplastic resin, the thermosetting resin, the rubber or the thermoplastic elastomer including the polyolefin-based elastomer blend, in which 0.1-30 parts by weight of calcium carbonate having an average particle diameter ranging from 5 to 10 nm is incorporated.

17. The method according to claim 13, **characterized in that** the marking portion comprises the thermoplastic resin, the thermosetting resin, the rubber or the thermoplastic elastomer including the polyolefin-based elastomer blend, in which 0.1-5 parts by weight of black iron oxide having an average particle diameter ranging from 0.3 to 0.8 µm is incorporated.

18. The method according to claim 13, **characterized in that** the marking portion comprises the thermoplastic resin, the thermosetting resin, the rubber or the thermoplastic elastomer including the polyolefin-based elastomer blend, in which 0.1-5 parts by weight of titanium black or titanium dioxide having an average particle diameter ranging from 0.1 to 0.8 µm is incorporated.

## Patentansprüche

1. Endoskop und medizinische Vorrichtung für ein Endoskop (3), wobei
das Endoskop und die medizinische Vorrichtung zumindest teilweise aus Thermoplast, Duroplast, Gummi oder aus thermoplastischem Elastomer geformt sind und einen Bereich aufweisen, der aus Thermoplast, Duroplast, Gummi oder aus thermoplastischem Elastomer geformt ist, von denen jeder/jedes 0,001 bis 20 Gewichtsteile Ruß mit einem durchschnittlichen Teilchendurchmesser von 10 bis 80 nm als Farbstoff/Farbentwicklungsmittel/Füllstoff enthält; und dass:
das Endoskop und die medizinische Vorrichtung einen Markierungsbereich (21) aufweisen, der Buchstaben und Symbole beinhaltende Markierungen angibt, die sich durch eine Bestrahlung des den Ruß enthaltenden Bereichs mit einem gepulsten YAG- oder YVO₄-Laserstrahl (2) mit einer Wellenlänge von 355, 532 oder 1064 nm entwickeln.

2. Endoskop und medizinische Vorrichtung für ein Endoskop nach Anspruch 1, **dadurch gekennzeichnet, dass** vorzugsweise der Ruß einen durchschnittlichen Teilchendurchmesser von 12 bis 40 nm aufweist.

3. Endoskop und medizinische Vorrichtung für ein Endoskop nach Anspruch 1, **dadurch gekennzeichnet, dass** der Markierungsbereich (21) Thermoplast, Duroplast, Gummi oder thermoplastisches Elastomer enthält, in die wenigstens eine Art von Material, ausgewählt aus der aus Ruß, Calciumcarbonat, Eisenoxidschwarz, Titanschwarz und Titanoxid bestehenden Gruppe, als Farbstoff/Farbentwicklungsmittel/Füllstoff eingebracht ist.

4. Endoskop und medizinische Vorrichtung für ein Endoskop nach Anspruch 1, **dadurch gekennzeichnet, dass** der Markierungsbereich eine Elastomermischung auf Polyolefin-Basis aufweist, die durch Vulkanisieren nur einer EPDM Gummiphase aus einem geschmolzenen PP/EPDM, der aus einer Mischung des Thermoplasts und des Gummis besteht, erhalten wird.

5. Endoskop und medizinische Vorrichtung für ein Endoskop nach Anspruch 1, **dadurch gekennzeichnet, dass** ein Hauptköper des Endoskops und der medizinischen Vorrichtung für das Endoskop einen in einen Patienten einzuführenden Einführabschnitt (11), einen Betätigungsabschnitt (12) zum Betätigen des Einführabschnitts (11) und einen Verbindungsabschnitt (13) aufweist, der zumindest mit einem Signalprozessor (8) zum Verarbeiten eines Bilds des Patienten zu verbinden ist; und
der Markierungsbereich (21) sowohl auf dem Einführabschnitt (11) als auch auf dem Betätigungsabschnitt (12) und auch auf dem Verbindungsabschnitt (13) ausgebildet ist.

6. Endoskop und medizinische Vorrichtung für ein Endoskop nach Anspruch 5, **dadurch gekennzeichnet, dass** der Markierungsbereich (21) auf einer Außenhaut (27) sowohl des Einführabschnitts (11) als auch des Betätigungsabschnitts (12) und auch des Verbindungsabschnitts (13) ausgebildet ist.

7. Endoskop und medizinische Vorrichtung für ein Endoskop nach Anspruch 4, **dadurch gekennzeichnet, dass** der Markierungsbereich (21) den Thermoplast, den Duroplast, den Gummi oder das thermoplastische Elastomer enthält, das die Elastomermischung auf Polyolefin-Basis beinhaltet, in die 0,1 bis 30 Gewichtsteile Calciumcarbonat mit einem durchschnittlichen Teilchendurchmesser von 5 bis 10 nm eingebracht sind.

8. Endoskop und medizinische Vorrichtung für ein Endoskop nach Anspruch 4, **dadurch gekennzeichnet, dass** der Markierungsbereich (21) den Thermoplast, den Duroplast, den Gummi oder das thermoplastische Elastomer enthält, das die Elastomermischung auf Polyolefin-Basis beinhaltet, in die 0,1 bis 5 Gewichtsteile Eisenoxidschwarz mit einem durchschnittlichen Teilchendurchmesser von 0,3 bis 0,8 µm eingebracht sind.

9. Endoskop und medizinische Vorrichtung für ein Endoskop nach Anspruch 4, **dadurch gekennzeichnet, dass** der Markierungsbereich (21) den Thermoplast, den Duroplast, den Gummi oder das thermoplastische Elastomer enthält, das die Elastomermischung auf Polyolefin-Basis beinhaltet, in die 0,1 bis 5 Gewichtsteile Titanschwarz oder Titanoxid mit einem durchschnittlichen Teilchendurchmesser von 0,1 bis 0,8 µm eingebracht sind.

10. Verfahren zum Aufbringen von Markierungen auf ein Endoskop und eine medizinische Vorrichtung für ein Endoskop (3), wobei
das Endoskop und die medizinische Vorrichtung zumindest teilweise aus Thermoplast, Duroplast, Gummi oder aus thermoplastischem Elastomer geformt sind und einen Bereich aufweisen, der aus dem Thermoplast, dem Duroplast, dem Gummi oder dem thermoplastischen Elastomer geformt ist, in die 0,001 bis 20 Gewichtsteile Ruß mit einem durchschnittlichen Teilchendurchmesser von 10 bis 80 nm als Farbstoff/Farbentwicklungsmittel/Füllstoff eingebracht sind;
der Bereich, in den der Ruß eingebracht ist, mit einem gepulsten YAG- oder YVO₄-Laserstrahl (2) mit einer Wellenlänge von 355, 532 oder 1064 nm bestrahlt wird, wodurch sich ein Markierungsbereich (21), der Buchstaben und Symbole beinhaltende Markierungen angibt, entwickelt.

11. Verfahren nach Anspruch 10, **dadurch gekennzeichnet, dass** der einen durchschnittlichen Teilchendurchmesser von vorzugsweise 12 bis 40 nm aufweisende Ruß in den Thermoplast, den Duroplast, den Gummi oder das thermoplastische Elastomer eingebracht ist.

12. Verfahren nach Anspruch 10, **dadurch gekennzeichnet, dass** wenigstens eine Art von Material, ausgewählt aus der aus Ruß, Calciumcarbonat, Eisenoxidschwarz, Titanschwarz und Titanoxid bestehenden Gruppe, als Farbstoff/Farbentwicklungsmittel/Füllstoff in den Thermoplast, den Duroplast, den Gummi oder das thermoplastische Elastomer eingebracht ist.

13. Verfahren nach Anspruch 10, **dadurch gekennzeichnet, dass** eine Elastomermischung auf Polyolefin-Basis, die durch Vulkanisieren nur einer EPDM Gummiphase aus einem geschmolzenen, aus einer Mischung des Thermoplasts und des Gummis bestehenden PP/EPDM erhalten wird, in den Bereich eingebracht wird, in dem die Farbentwicklung stattzufinden hat.

14. Verfahren nach Anspruch 10, **dadurch gekennzeichnet, dass** ein Hauptköper des Endoskops und der medizinischen Vorrichtung für das Endoskop einen in einen Patienten einzuführenden Einführabschnitt (11), einen Betätigungsabschnitt (12) zum Betätigen des Einführabschnitts (11) und einen Verbindungsabschnitt (13) aufweist, der zumindest mit einem Signalprozessor (8) zum Verarbeiten eines Bilds des Patienten zu verbinden ist; und wobei
die die Buchstaben und Symbole beinhaltende Markierung sowohl auf dem Einführabschnitt (11) als auch auf dem Betätigungsabschnitt (12) und auch auf dem Verbindungsabschnitt (13) des Hauptkörpers des Endoskops ausgebildet ist.

15. Verfahren nach Anspruch 14, **dadurch gekennzeichnet, dass** die die Buchstaben und Symbole beinhaltende Markierung auf der Außenhaut sowohl des Einführabschnitts als auch des Betätigungsabschnitts und auch des Verbindungsabschnitts des Hauptkörpers des Endoskops ausgebildet ist.

16. Verfahren nach Anspruch 13, **dadurch gekennzeichnet, dass** der Markierungsbereich den Thermoplast, den Duroplast, den Gummi oder das thermoplastische Elastomer enthält, das die Elastomermischung auf Polyolefin-Basis beinhaltet, in die 0,1 bis 30 Gewichtsteile Calciumcarbonat mit einem durchschnittlichen Teilchendurchmesser von 5 bis 10 nm eingebracht sind.

17. Verfahren nach Anspruch 13, **dadurch gekennzeichnet, dass** der Markierungsbereich den Thermoplast, den Duroplast, den Gummi oder das thermoplastische Elastomer enthält, das die Elastomermischung auf Polyolefin-Basis beinhaltet, in die 0,1 bis 5 Gewichtsteile Eisenoxidschwarz mit einem durchschnittlichen Teilchendurchmesser von 0,3 bis 0,8 µm eingebracht sind.

18. Verfahren nach Anspruch 13, **dadurch gekennzeichnet, dass** der Markierungsbereich den Thermoplast, den Duroplast, den Gummi oder das thermoplastische Elastomer enthält, das die Elastomermischung auf Polyolefin-Basis beinhaltet, in die 0,1 bis 5 Gewichtsteile Titanschwarz oder Titanoxid mit einem durchschnittlichen Teilchendurchmesser von 0,1 bis 0,8 µm eingebracht sind.

## Revendications

1. Endoscope et instrument médical pour un endoscope (3), dans lesquels :
l'endoscope et l'instrument médical sont au moins partiellement constitués d'une résine thermoplastique, d'une résine thermodurcissable, d'un caoutchouc ou d'un élastomère thermoplastique et sont prévus avec une partie qui est constituée d'une résine thermoplastique, d'une résine thermodurcissable, d'un caoutchouc ou d'un élastomère thermoplastique contenant chacun 0,001 à 20 partie(s) en poids d'un noir de carbone ayant un diamètre moyen de particules de 10 à 80 nm comme une matière colorante/un agent de révélation de couleur/une charge ; et
l'endoscope et l'instrument médical sont prévus avec une partie (21) de marquage indiquant des marques incluant des lettres et des symboles qui sont révélées par l'intermédiaire d'une projection sur la partie contenant le noir de carbone d'un faisceau laser pulsé (2) de YAG ou YVO₄ ayant une longueur d'onde de 355, 532 ou 1 064 nm.

2. Endoscope et instrument médical pour endoscope selon la revendication 1, **caractérisés en ce que**, préférablement, le noir de carbone a un diamètre moyen de particules de 12 à 40 nm.

3. Endoscope et instrument médical pour endoscope selon la revendication 1, **caractérisés en ce que** la partie (21) de marquage comprend une résine thermoplastique, une résine thermodurcissable, un caoutchouc ou un élastomère thermoplastique dans lequel/laquelle au moins un type de matière choisie parmi le groupe consistant en un noir de carbone, un carbonate de calcium, un oxyde ferrosoferrique, un noir de titane et un dioxyde de titane est incorporée comme une matière colorante/un agent de révélation de couleur/une charge.

4. Endoscope et instrument médical pour endoscope selon la revendication 1, **caractérisés en ce que** la partie de marquage comprend un mélange élastomérique à base de polyoléfine qui est obtenu par la vulcanisation de seulement une phase caoutchouc d'EPDM d'un PP/EPDM fusionné constitué d'un mélange de la résine thermoplastique et du caoutchouc.

5. Endoscope et instrument médical pour endoscope selon la revendication 1, **caractérisés en ce qu'**un corps principal de l'endoscope et de l'instrument médical pour endoscope comprend une partie (11) d'insertion destinée à être insérée dans un sujet, une partie (12) de manipulation pour manipuler la partie (11) d'insertion, et une partie (13) de connecteur destinée à être connectée au moins avec un processeur (8) de signaux pour traiter une image du sujet ; et
la partie (21) de marquage est formée au niveau de chacune parmi la partie (11) d'insertion, la partie (12) de manipulation et la partie (13) de connecteur.

6. Endoscope et instrument médical pour endoscope selon la revendication 5, **caractérisés en ce que** la partie (21) de marquage est formée sur une peau (27) de chacune parmi la partie (11) d'insertion, la partie (12) de manipulation et la partie (13) de connecteur.

7. Endoscope et instrument médical pour endoscope selon la revendication 4, **caractérisés en ce que** la partie (21) de marquage comprend la résine thermoplastique, la résine thermodurcissable, le caoutchouc ou l'élastomère thermoplastique incluant le mélange élastomérique à base de polyoléfine, dans lequel 0,1 à 30 parties en poids de carbonate de calcium ayant un diamètre moyen de particules dans une plage de 5 à 10 nm est/sont incorporée(s).

8. Endoscope et instrument médical pour endoscope selon la revendication 4, **caractérisés en ce que** la partie (21) de marquage comprend la résine thermoplastique, la résine thermodurcissable, le caoutchouc ou l'élastomère thermoplastique incluant le mélange élastomérique à base de polyoléfine, dans lequel 0,1 à 5 parties en poids d'oxyde ferrosoferrique ayant un diamètre moyen de particules dans une plage de 0,3 à 0,8 µm est/sont incorporée(s).

9. Endoscope et instrument médical pour endoscope selon la revendication 4, **caractérisés en ce que** la partie (21) de marquage comprend la résine thermoplastique, la résine thermodurcissable, le caoutchouc ou l'élastomère thermoplastique incluant le mélange élastomérique à base de polyoléfine, dans lequel 0,1 à 5 parties en poids de noir de titane ou de dioxyde de titane ayant un diamètre moyen de particules dans une plage de 0,1 à 0,8 µm est/sont incorporée(s).

10. Procédé d'application de marquages à un endoscope et un instrument médical pour endoscope (3), dans lequel
l'endoscope et l'instrument médical sont au moins partiellement constitués d'une résine thermoplastique, d'une résine thermodurcissable, d'un caoutchouc ou d'un élastomère thermoplastique et sont prévus avec une partie qui est constituée de la résine thermoplastique, de la résine thermodurcissable, du caoutchouc ou de l'élastomère thermoplastique dans lequel/laquelle 0,001 à 20 partie(s) en poids d'un noir de carbone ayant un diamètre moyen de particules de 10 à 80 nm est/sont incorporée(s) comme un(e) matière colorante/agent de révélation de couleur/charge ;
la partie contenant le noir de carbone reçoit un faisceau laser pulsé (2) de YAG ou YVO₄ ayant une longueur d'onde de 355, 532 ou 1 064 nm, révélant ainsi une partie (21) de marquage indiquant des marques incluant des lettres et des symboles.

11. Procédé selon la revendication 10,
**caractérisé en ce que** le noir de carbone ayant un diamètre moyen de particules, préférablement, dans une plage de 12 à 40 nm est incorporé dans la résine thermoplastique, la résine thermodurcissable, le caoutchouc ou l'élastomère thermoplastique.

12. Procédé selon la revendication 10, **caractérisé en ce qu'**au moins un type de matière choisie parmi le groupe consistant en un noir de carbone, un carbonate de calcium, un oxyde ferrosoferrique, un noir de titane et un dioxyde de titane est incorporée, comme une matière colorante/un agent de révélation de couleur/une charge, dans la résine thermoplastique, la résine thermodurcissable, le caoutchouc ou l'élastomère thermoplastique.

13. Procédé selon la revendication 10, **caractérisé en ce qu'**un mélange élastomérique à base de polyoléfine qui est obtenu par la vulcanisation de seulement une phase caoutchouc d'EPDM d'un PP/EPDM fusionné constitué d'un mélange de la résine thermoplastique et du caoutchouc est incorporé dans la partie dont la couleur doit être révélée.

14. Procédé selon la revendication 10, **caractérisé en ce qu'**un corps principal de l'endoscope et de l'instrument médical pour endoscope comprend une partie (11) d'insertion destinée à être insérée dans un sujet, une partie (12) de manipulation pour manipuler la partie d'insertion, et une partie (13) de connecteur destinée à être connectée au moins avec un processeur (8) de signaux pour traiter une image du sujet ; et dans lequel
le marquage incluant les lettres et les symboles est formé au niveau de chacune parmi la partie (11) d'insertion, la partie (12) de manipulation et la partie (13) de connecteur du corps principal de l'endoscope.

15. Procédé selon la revendication 14, **caractérisé en ce que** le marquage incluant les lettres et les symboles est formé au niveau d'une peau de chacune parmi la partie d'insertion, la partie de manipulation et la partie de connecteur du corps principal de l'endoscope.

16. Procédé selon la revendication 13, **caractérisé en ce que** la partie de marquage comprend la résine thermoplastique, la résine thermodurcissable, le caoutchouc ou l'élastomère thermoplastique incluant le mélange élastomérique à base de polyoléfine, dans lequel 0,1 à 30 parties en poids de carbonate de calcium ayant un diamètre moyen de particules dans une plage de 5 à 10 nm est/sont incorporée(s).

17. Procédé selon la revendication 13, **caractérisé en ce que** la partie de marquage comprend la résine thermoplastique, la résine thermodurcissable, le caoutchouc ou l'élastomère thermoplastique incluant le mélange élastomérique à base de polyoléfine, dans lequel 0,1 à 5 parties en poids d'oxyde ferrosoferrique ayant un diamètre moyen de particules dans une plage de 0,3 à 0,8 µm est/sont incorporée(s).

18. Procédé selon la revendication 13, **caractérisé en ce que** la partie de marquage comprend la résine thermoplastique, la résine thermodurcissable, le caoutchouc ou l'élastomère thermoplastique incluant le mélange élastomérique à base de polyoléfine, dans lequel 0,1 à 5 parties en poids de noir de titane ou de dioxyde de titane ayant un diamètre moyen de particules dans une plage de 0,1 à 0,8 µm est/sont incorporée(s).
